(19) Europäisches Patentamt
European Patent Office
Office européen des brevets

(11) Publication number: **0 104 959**
**B1**

# (12) EUROPEAN PATENT SPECIFICATION

(45) Date of publication of patent specification: **21.01.87**

(21) Application number: **83401199.1**

(22) Date of filing: **10.06.83**

(51) Int. Cl.[4]: **C 07 D 215/48**, C 07 D 401/04, C 07 D 405/04, C 07 D 409/04, A 61 K 31/47

## (54) 4-Quinolone derivatives.

(30) Priority: **14.06.82 JP 102592/82**

(43) Date of publication of application: **04.04.84 Bulletin 84/14**

(45) Publication of the grant of the patent: **21.01.87 Bulletin 87/04**

(84) Designated Contracting States: **CH DE FR GB IT LI**

(56) References cited:
**EP-A-0 062 001**
**CH-A- 560 193**
**DE-A-2 910 195**
**GB-A-1 398 066**
**US-A-3 960 868**

(73) Proprietor: **NIPPON SHINYAKU COMPANY, LIMITED**
**14, Kisshoin Nishinosho Monguchicho**
**Minami-ku Kyoto-shi Kyoto (JP)**

(72) Inventor: **Enomoto, Hiroshi**
**26-3-7-707 Baba Miba Hashiri**
**Nagaokakyo-shi, Kyoto-fu 617 (JP)**
Inventor: **Nomura, Tadatoshi**
**39-905 Okurayama Kobata**
**Uji-shi Kyoto-fu 611 (JP)**
Inventor: **Aoyagi, Yoshiaki**
**8-7-105 Uchidehama**
**Otsu-shi Shiga-ken 520 (JP)**
Inventor: **Chokai, Shoichi**
**1-57 Yanamoto Nakaaraita Sasa-cho**
**Kameoka-shi Kyoto-fu 621 (JP)**
Inventor: **Fujita, Yukio**
**489 Nariai**
**Takatsuki-shi Osada-fu 569 (JP)**
Inventor: **Kono, Tatsuhiko**
**F-306 27 Shinashiyaue**
**Suita-shi Osaka-fu 565 (JP)**
Inventor: **Murase, Masao**
**439-11 Nomuracho**
**Kasatsu-shi Shiga-ken 515 (JP)**
Inventor: **Inoue, Kichiro**
**346-12 Ninomarucho Mukaijima**
**Fushimi-ku Kyoto 612 (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 104 959 B1**

Inventor: **Adachi, Masahiro**
**6-1 Nagaodai-2-chome**
**Hirakata-shi Osaka-fu 573-01 (JP)**

(74) Representative: **Hranitzky, Wilhelm Max et al**
**NOVAPAT - CABINET CHEREAU 5, Place du Molard**
**CH-1204 Genève (CH)**

**Description**

The present invention relates to 4-quinolone derivatives represented by one of the following general formulae (Ia), (Ib) and (Ic) and salts thereof, as well as their use as pharmaceuticals and methods for manufacturing them:

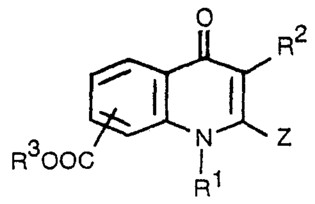

(Ia)

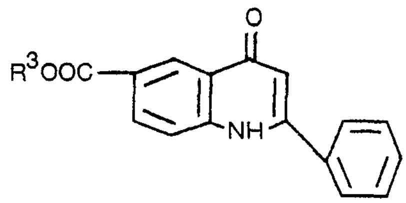

(Ib)

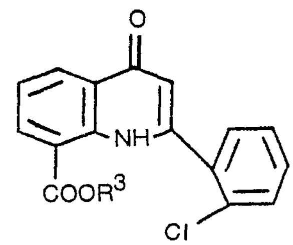

(Ic)

In the formula (Ia),

$R^1$ and $R^2$ are same or different and they are hydrogen, lower alkyl of one to eight carbon chain length or lower alkenyl of up to four carbon chain length; $R^3$ is hydrogen, lower alkyl of 1 to 8 carbon chain length, lower alkyl of 1 to carbon chain length with 1 or 2 hydroxyl group(s), lower alkyl of 3 to 10 carbon chain length with 1 or 2 ether bond(s) or $-(CH_2)_nA$ where n is an integer of 1 to 3 and A is acetyl, acetyloxy, cyano or phenoxy;

Z is phenyl with or without substituent(s) selected from a group consisting of hydrogen, halogen, lower alkyl of 1 to 8 carbon chain length, lower haloalkyl of 1 to 3 carbon chain length, lower alkoxy or 1 to 4 carbon chain length and $COOR^4$ in which $R^4$ is hydrogen or lower alkyl of 1 to 4 carbon chain length, or Z may be five- or six-membered unsaturated hetero ring radical having nitrogen, oxygen or sulfur as hetero-atom;

in the formula (Ib), $R^3$ is phenethyl, and, in the formula (Ic), $R^3$ is allyl, ethoxycarbonylmethyl, ethoxy-carbonylpropyl, betahydroxyethoxyethyl or ethoxycarbonylacetylmethyl.

The present invention compounds exhibit anti-allergic action, expectorant action, inhibiting action against coagulation of platelet and are useful as medicaments such as anti-allergic agent, antitussives, expectorants, antithrombotic agents, remedies for asthma and the like.

The compounds of this type exhibit marked characteristics such that they can be administered orally while conventional similar agents do not and further that their pharmaceutical actions are long acting.

For instance, sodium cromoglicate recently developed is reported by Cox, et al to be effective against allergic asthma (cf. Advances in Drug Research, volume 5, page 115, 1970). It is supposed that this compound inhibits emission of chemical mediators from mast cells. Unfortunately this compound has a disadvantage in that it does not show any pharmaceutical effect by oral administration and furthermore that the duration of its action is short.

Recently it has been known that SRS—A (slow reacting substance of anaphylaxis) which is one of the chemical mediators, plays a main role at the onset of asthma and, under such a situation, development of new and specific pharmaceuticals antagonizing against the action of SRS—A and exhibiting biosynthesis inhibiting action has been expected.

In view of the above, the present inventors have conducted extensive studies in order to find pharmaceuticals having SRS—A antagonizing action and synthesis inhibiting action and these have resulted in the present invention.

Examples of lower alkyls in the present invention are methyl, ethyl, propyl, isopropyl, butyl, pentyl, hexyl, heptyl, octyl and the like. Examples of lower alkoxy are methoxy, ethoxy, propoxy, isopropoxy, butoxy, tertiary butoxy, and the like. Examples of alkenyls are vinyl, allyl, and the like. Examples of aralkyls are benzyl, phenethyl and the like. The term halogen stands for fluorine, chlorine, bromine and iodine.

Examples of substituents represented by Z are, besides phenyl, such as 2-fluorophenyl, 2-chlorophenyl, 3-chlorophenyl, 4-chlorophenyl, 2-bromophenyl, 2-iodophenyl, 2,4-dichlorophenyl, 3,4-dichlorophenyl, 2,5-dichlorophenyl, o-tolyl, p-tolyl, 2-ethylphenyl, 4-isopropylphenyl, 4-pentylphenyl, 2,4-dimethylphenyl, 3-trifluoromethylphenyl, 2-methoxyphenyl, 4-methoxyphenyl, 2-ethoxyphenyl, 2-isopropoxyphenyl, 2,4-dimethoxyphenyl, 3,4-dimethoxyphenyl, 4-carboxyphenyl, 4-methoxycarbonylphenyl and the like and further examples are pyrrolyl, pyrrolinyl, pyridyl, furyl, thienyl and the like.

For the sake of convenience, the compound (Ia) is represented as the 4-quinolone type but, needless to say, it also includes the following tautomers:

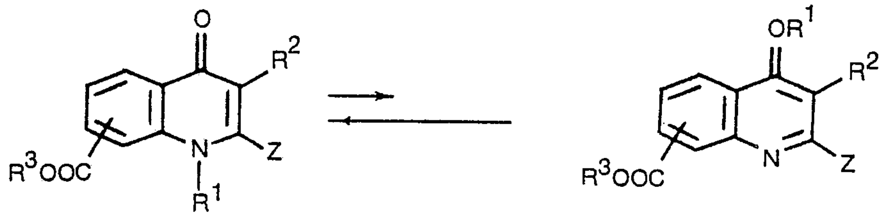

(Ia) (I′)

4-Quinolone derivatives according to the present invention are prepared by subjecting compounds (II) represented by the following formula or tautomers thereof

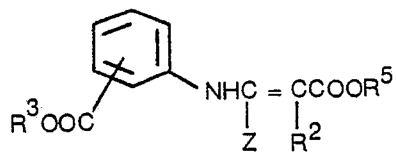

(II)

(in which the meanings of $R^2$, $R^3$ and Z are the same as already defined and $R^5$ stands for lower alkyl such as, for example, methyl, ethyl, propyl and the like to a ring closure reaction.

The reaction is conducted by heating the reactants in a suitable solvent (high boiling solvents such as dichlorobenzene, tetraline, diphenyl ether, diethylene glycol, dimethyl ether and the like) for a period of from thirty minutes to ten hours.

The compounds (Ia) in which the radical $R^1$ is lower alkyl or alkenyl can be prepared by reacting the 4-quinolone derivatives obtained by the above method with lower alkyl halide, dialkyl sulfate or alkenyl halide in the presence of suitable base or acid-removing agent. The alkyl halides used for alkylating in the above reaction are, for example, methyl iodide, ethyl iodide, methyl bromide, ethyl bromide, propyl bromide, butyl bromide and the like. Examples of dialkyl sulfate applicable are dimethyl sulfate, diethyl sulfate and the like. Examples of alkenyl halide used for alkenylation are vinyl bromide, allyl bromide and the like. Examples of bases or acid removing agents applicable are sodium carbonate, potassium carbonate, sodium bicarbonate, potassium bicarbonate, sodium hydroxide, potassium hydroxide, sodium alkoxides, sodium hydride and the like.

The reaction is carried out by heating the reactants in a suitable solvent (such as, for example, tetrahydrofuran, dioxane, toluene, xylene, dimethyl formamide, dimethyl sulfoxide and the like) at 50 to 120°C within a range of thirty minutes to ten hours.

Compounds represented by the formula (II) can be obtained by a condensation of a compound represented by the formula (III).

$$\underset{\displaystyle R^2}{ZCOCHOOR^5} \qquad \text{(III)}$$

(in which the definitions for Z, $R^2$ and $R^5$ are the same as already defined) with aminobenzoate represented by the formula (IV)

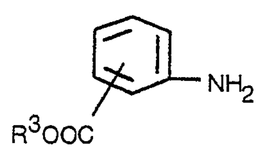

(IV)

(in which the meaning of $R^3$ is the same as already defined). The reaction is conducted by heating the reactants in a suitable solvent (such as benzene, toluene, chloroform and the like) in the presence of catalytic amount of acid (such as p-toluenesulfonic acid, benzenesulfonic acid, sulfuric acid, hydrochloric acid and the like) for a period of one day to three days followed by dehydrating using a Dienstag apparatus or by heating to reflux for one to three days in ethanol in the presence of activated anhydrous calcium sulfate.

The compounds (III) in which $R^2$ is lower alkyl or alkenyl can be prepared by the reaction of the compound of a general formula (V)

$$ZCOCH_2COOR^5 \qquad \text{(V)}$$

(in which meanings of Z and $R^5$ are the same as already defined) with alkyl halide or with alkenyl halide in the presence of a base. The reaction is carried out by stirring the reactants in a suitable solvent (such as, for example, benzene, toluene, tetrahydrofuran, methanol, ethanol, dimethyl formamide, acetonitrile and the like) at a temperature range of −5°C to 80°C for a time range of thirty minutes to five hours. Examples of bases used are sodium hydride, sodium alkoxides, and the like and examples of alkyl halides are methyl iodide, ethyl iodide, methyl bromide, ethyl bromide, propyl bromide, butyl bromide and the like. Alternatively, examples of alkenyl halides are vinyl bromide, allyl bromide, and the like. Benzoyl (heterocyclic carbonyl) acetate derivatives (V) used as starting materials in the above reaction are partly known and partly new compounds.

Compounds represented by the general formula (V) are prepared as follows. Thus, acetyl compounds represented by the general formula (VI)

$$ZCOCH_3 \qquad (VI)$$

(in which the meaning of Z is the same as already defined) are subjected to a reaction with dialkyl carbonates (such as dimethyl carbonate, diethyl carbonate and the like) in a suitable solvent (such as ether, tetrahydrofuran and the like) in the presence of bases (such as sodium hydride, sodium amide and the like). Alternatively, acetates are made to react with n-butyl lithium in the presence of cyclohexyl isopropylamine followed by a reaction with acid chlorides of a general formula (VII)

$$ZCOCl \qquad (VII)$$

(in which the meaning of Z is the same as already defined) in a suitable solvent such as ether, tetrahydrofuran and the like.

Examples of other synthetic methods are as follows. Thus acetoacetate is made to react with an acid chloride of the formula (VII) in the presence of a base in a suitable solvent such as water or tetrahydrofuran and then deacetylated with ammonium chloride. Or acetoacetate in a form of alkali metal salt is made to react with benzoate followed by deacetylation.

4-Quinolone derivatives prepared by the above methods can be easily isolated and purified by conventional routes such as, for example, recrystallization, chromatography and the like.

Among the present invention compounds prepared as above, those in which $R^3$ is hydrogen can be easily made into salts with conventional pharmaceutically acceptable basic compounds. Examples of such basic substances are sodium hydroxide, potassium hydroxide, potassium aluminium hydroxide, calcium hydroxide, sodium carbonate, potassium carbonate, sodium bicarbonate, potassium bicarbonate and other inorganic basic compounds as well as organic basic compounds such as morpholine, piperazine, thiomorpholine, triethylamine and the like. The salts may also include salts with mineral acids or organic acids where possible. Example of such salts are ethyl 2-(2-chlorophenyl)-4-hydroxyquinoline-8-carboxylate hydrochloride.

An illustration of such synthetic routes is as follows:

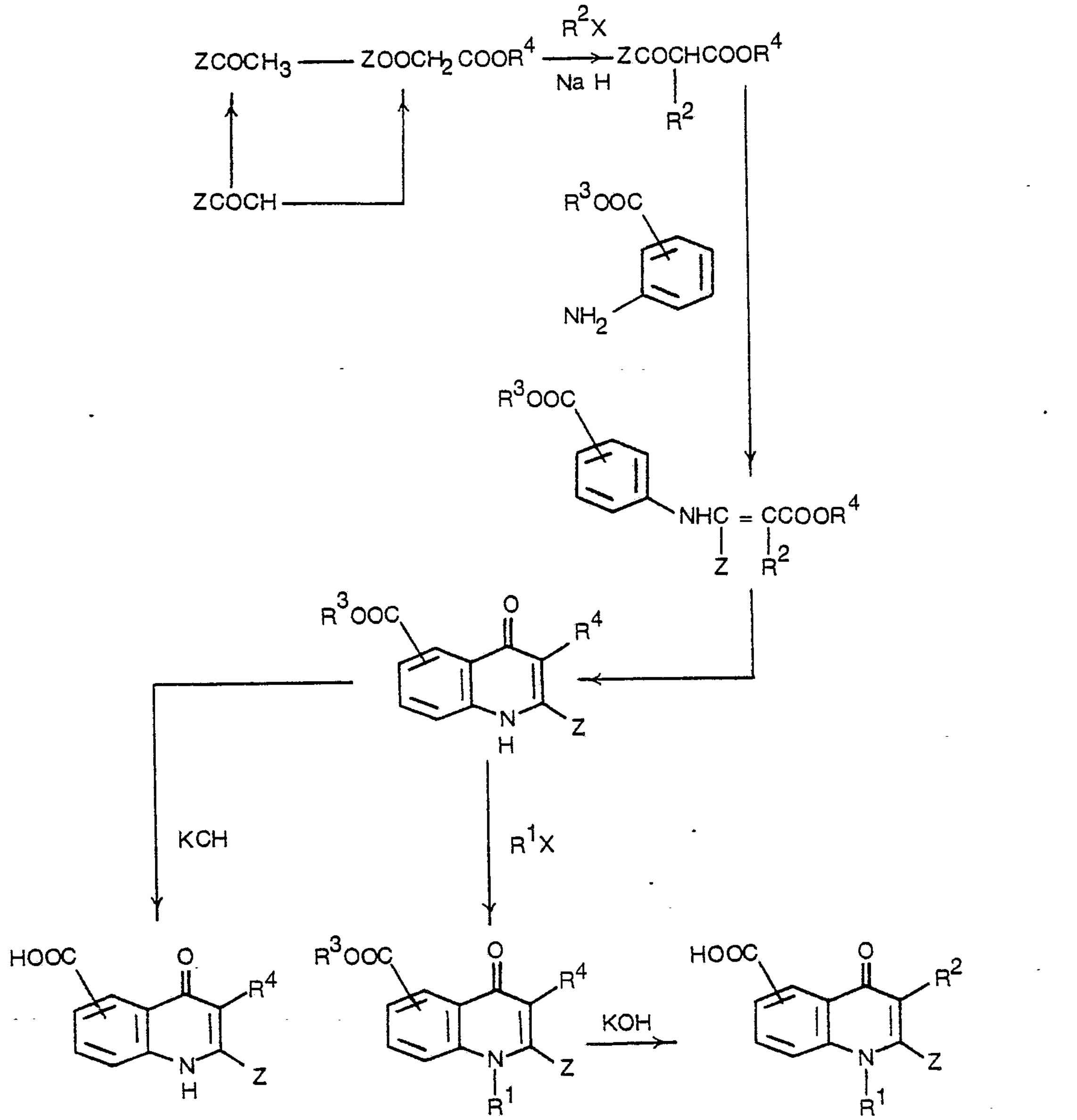


Alternatively, compounds represented by the general formula (I) may also be prepared by the following routes:

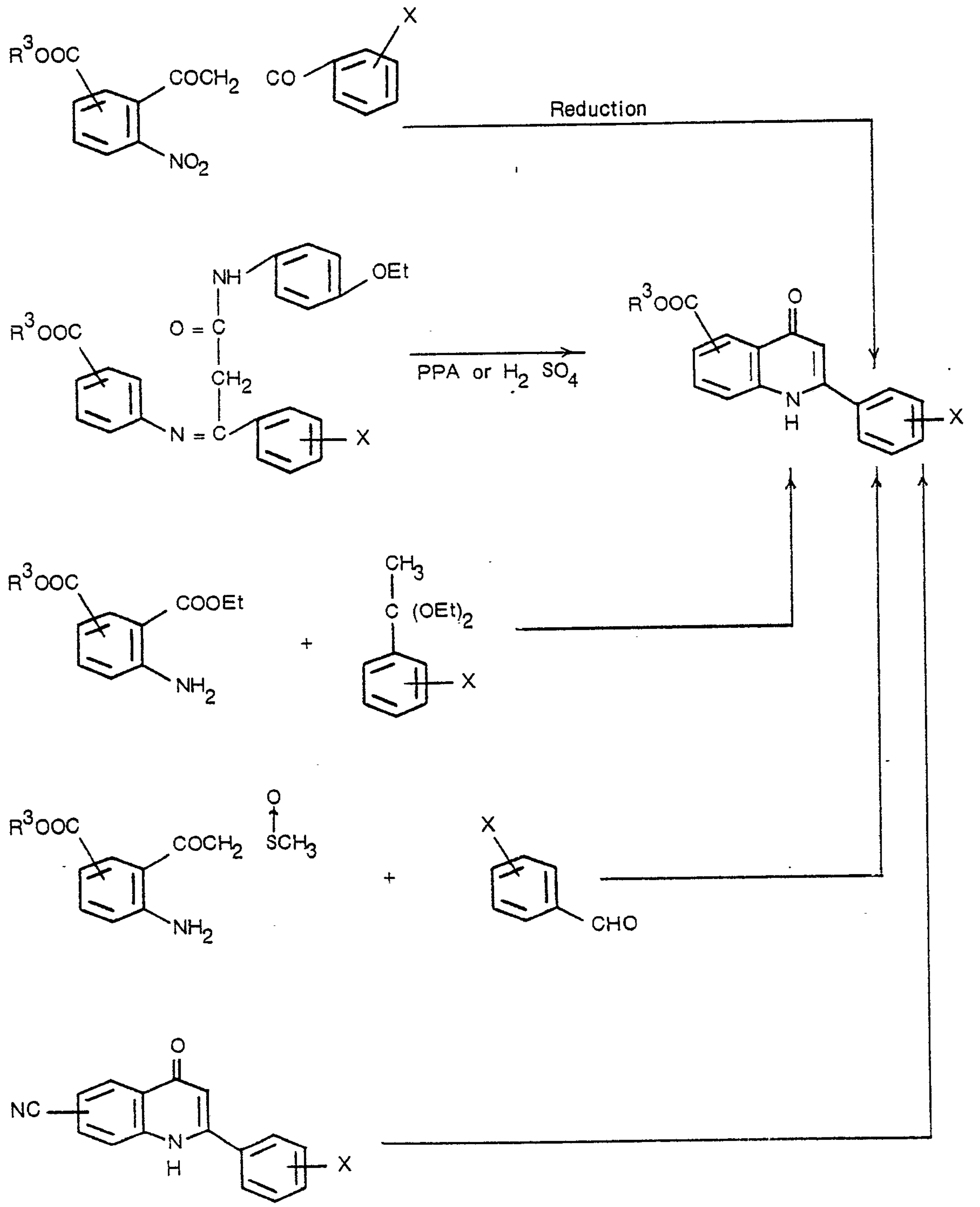


(I)

The compounds of the present invention exhibit antiallergic action and, accordingly, it is possible that they can be administered to patients suffering from asthma, hay fever (nose allergy), hives, atopic dermatitis and the like. In addition, the compounds exhibit antiinflammatory action and, therefore, they can be given to patients suffering from chronic articular rheumatism, pain after operation, arthrosis deformans, low back pain, acute upper respiratory inflammation, toothache and dysmenorrhea, and the like. They also inhibit the platelet coagulation and may be effective against various diseases caused by rise in platelet coagulation.

Effects of the present invention compounds as remedies for allergic symptoms were evaluated by a passive cutaneous anaphylaxie assay (PCA) in rats and by measuring anti-SRS—A action using ileus of guinea pigs.

Test method No. 1 (PCA)

(i) Antiserum abundant in homocytotropic antibody is prepared by the same method as Tada and

Okumura did (cf. Journal of Immunology, volume 106, page 1002, 1971). Thus, 1 mg (calculated as an amount of protein) of DNP-As (2,4-dinitrophenyl-coupled ascaris extract) prepared by methods of Strejan and Campbell (Journal of Immunology, volume 98, page 893, 1967) and of Eisen (Journal of American Chemical Society, volume 75, page 4593, 1953) and $1 \times 10^{10}$ pertussis vaccine are administered to each paw of Wistar strain rats (180 to 200 grams body weights) by dividing the dose by four. Five days later 0.5 mg of DNP-As is administered into muscle of back. Eight days later from the initial immunization, blood is taken from descending aorta under anesthetizing with ether, the resulting serum is stored at −80° and is melted before use.

(ii) Effect of tested compounds is investigated as follows:

Anti-serum obtained by the method (i) is diluted with physiological saline solution double by double successively and 0.05 ml of each diluted solution is administered into the back of Wistar strain rats (140 to 160 grams body weight) intradermally. After 72 hours, a solution of 2 mg calculated as protein of DNP-As and 2.5 mg of Evans Blue dissolved in 1 ml of physiological saline solution is administered intravenously at a dose of 5 ml/kg. After 30 minutes from antigen solution administration, the animals are killed and diameters of blue spots appeared at the place where antiserum is administered are measured. The PCA test is conducted by the same method as already described using a diluted solution of antiserum which always show 10 mm or more spot diameters and the effect of the test compounds is judged. Thus antiserum diluted solutions are administered to two places in back. Test compounds are administered orally at the dose of 10 mg/kg one hour before administration of antigen solution. From the skin of reacted parts of killed animals, leaked or emitted dyestuff is extracted and the amount of the dyestuff is measured. The inhibition ratio is calculated by the following expression:

$$\text{Inhibition Ratio} = \left(1 - \frac{A'}{A}\right) \times 100$$

in which A' is an amount of dyestuff in the group treated with the test compounds and A is that in the control group.

Test Method No. 2

Anti-SRS-A action (An anti-action against slow reacting substance of anaphylaxis).

Hartley strain male guinea pigs (300 to 350 grams body weight) are killed and 1.0 to 1.5 cm of ileum is immediately excised from ileocecal parts and is suspended in 10 ml of Tyrode solution (95% $O_2$ — 5% $CO_2$ saturation) containing $10^{-7}$ g/ml of atropine and $10^{-6}$ g/ml of pyrilamine. SRS—A (20 units) (the amount of SRS—A showing the same contraction as 5 ng of histamine is defined as 1 unit) prepared by using sensitized guinea pig lung is given to cause contraction there. Then antagonistic action of test compounds treated five minutes ago against the shrinkage is measured and recorded via isotonic transducer.

$$\text{Inhibition Ratio of Test Compd (\%)} = \left(1 - \frac{A'}{A}\right) \times 100$$

in which A' is a height of contraction of SRS—A + test compound and A is that of SRS—A.

TABLE 1

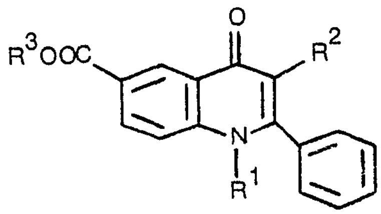

| R¹ | R² | R³ | PCA Inhibition Ratio% | 1/1000mM SRS—A Inhibition Ratio% |
|---|---|---|---|---|
| H | H | H | 22.5 | 23.9 |
| H | H | Na salt | 27.9 | 35.0 |
| H | H | Methyl | 25.4 | 77.0 |
| H | H | Ethyl | 25.7 | 27.5 |
| H | H | iso-Propyl | 26.2 | 36.7 |
| H | H | Phenethyl | 24.3 | 22.3 |
| H | Methyl | H | 22.4 | 21.0 |
| H | Methyl | Methyl | 26.3 | 54.8 |
| H | Allyl | H | 17.5 | 22.3 |
| H | Allyl | Methyl | 20.1 | 37.5 |
| Methyl | H | H | 16.2 | 22.0 |
| Methyl | H | Methyl | 21.3 | 100 |
| Ethyl | H | H | 16.4 | 28.9 |
| Ethyl | H | Methyl | 18.9 | 25.4 |
| n-Propyl | H | H | 23.4 | 19.7 |
| n-Propyl | H | Methyl | 19.7 | 65.3 |
| Allyl | H | H | 26.5 | 24.3 |
| Allyl | H | Methyl | 20.4 | 22.7 |
| n-Hexyl | H | H | 26.4 | 83.9 |
| n-Hexyl | H | Methyl | 27 | 27.5 |

In case Z is substituted phenyl — 1:

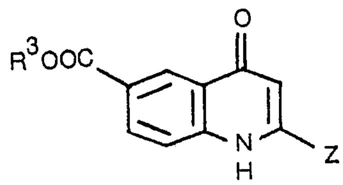

| Substituent on Phenyl | $R^3$ | PCA Inhibition Ratio% | 1/1000mM SRS—A Inhibition Ratio% |
|---|---|---|---|
| 2-Chloro | Methyl | 7.8 | 48.3 |
| 2-Chloro | H | 35.8 | 26.4 |
| 2-Chloro | Na salt | 18.7 | 24.9 |
| 2-Bromo | Methyl | 23.4 | 55.8 |
| 2-Bromo | H | 17.2 | 25.7 |
| 2-Fluoro | Methyl | 20.6 | 38.5 |
| 2-Fluoro | H | 25.6 | 23.4 |
| 2-Iodo | Methyl | 19.6 | 55.6 |
| 3-Chloro | Methyl | 21.2 | 33.3 |
| 3-Chloro | H | 43.0 | 24.5 |
| 3-Chloro | Na salt | 24.4 | 25.5 |
| 4-Chloro | Methyl | 24.4 | 20.6 |
| 4-Chloro | H | 22.3 | 22.5 |
| 2,4-Dichloro | Methyl | 17.4 | 19.8 |
| 2,4-Dichloro | H | 39.4 | 25.6 |
| 2,4-Dichloro | Na salt | 20.4 | 14.5 |
| 3,4-Dichloro | Methyl | 17.1 | 24.3 |
| 3,4-Dichloro | H | 20.4 | 20.5 |
| 2,5-Dichloro | Methyl | 24.8 | 66.0 |
| 2,5-Dichloro | H | 25.4 | 27.5 |
| 2-Methyl | Methyl | 29.5 | 29.1 |
| 2-Methyl | H | 43.2 | 17.4 |
| 2-Methyl | Na salt | 24.8 | 17.3 |
| 2-Ethyl | Methyl | 18.0 | 41.8 |
| 2-Ethyl | H | 24.2 | 25.4 |

| Substituent on Phenyl | $R^3$ | PCA Inhibition Ratio% | 1/1000mM SRS—A Inhibition Ratio% |
|---|---|---|---|
| 4-Methyl | Methyl | 27.3 | 19.8 |
| 4-Methyl | H | 17.3 | 18.9 |
| 4-Isopropyl | Methyl | 26.4 | 24.2 |
| 4-Isopropyl | H | 25.2 | 18.8 |
| 4-n-Pentyl | Methyl | 19.4 | 36.0 |
| 4-n-Pentyl | H | 22.7 | 61.4 |
| 3-Trifluoromethyl | Methyl | 25.0 | 21.3 |
| 3-Trifluoromethyl | H | 22.4 | 30.0 |
| 2,4-Dimethyl | Methyl | 18.9 | 22.4 |
| 2,4-Dimethyl | H | 16.8 | 17.5 |
| 2-Methoxy | Methyl | 22.3 | 36.2 |
| 2-Methoxy | H | 20.4 | 27.7 |
| 2-Ethoxy | Methyl | 23.1 | 63.0 |
| 2-Ethoxy | H | 15.8 | 24.6 |
| 2-Isopropoxy | Methyl | 21.2 | 58.8 |
| 2-Isopropoxy | H | 20.1 | 31.8 |
| 4-Methoxy | Methyl | 19.0 | 27.4 |
| 4-Methoxy | H | 18.4 | 23.0 |
| 2,4-Dimethoxy | Methyl | 17.7 | 48.2 |
| 2,4-Dimethoxy | H | 26.9 | 27.3 |
| 3,4-Dimethoxy | Methyl | 25.4 | 25.7 |
| 3,4-Dimethoxy | H | 17.4 | 25.0 |
| 4-Methoxycarbonyl | Methyl | 19.8 | 36.8 |
| 4-Carboxy | H | 25.8 | 27.9 |

In case where Z is heterocyclic ring:

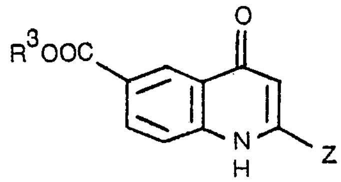

| (Z) | | | |
|---|---|---|---|
| 4-Pyridyl | Methyl | 22.3 | 21.4 |
| 4-Pyridyl | H | 25.6 | 25.0 |
| 3-Pyridyl | Methyl | 17.8 | 22.0 |
| 3-Pyridyl | H | 20.4 | 23.3 |
| 2-Pyridyl | Methyl | 23.1 | 25.0 |
| 2-Pyridyl | H | 20.4 | 28.9 |
| 2-Furyl | Methyl | 21.3 | 25.6 |
| 2-Furyl | H | 17.4 | 21.0 |
| 2-Thienyl | Methyl | 26.7 | 23.3 |
| 2-Thienyl | H | 19.8 | 19.5 |

In case where Z is substituted phenyl — 2;

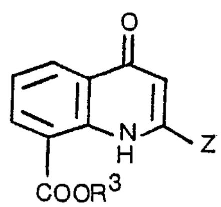

| (Substituent on Phenyl) | | | |
|---|---|---|---|
| H | H | 35.3 | 26.4 |
| H | Na salt | 10.0 | 27.3 |
| H | Ethyl | 20.9 | 52.4 |
| 2-Chlor | Ethyl | 28.5 | 75.9 |
| 2-Chloro | H | 17.0 | 27.9 |
| 2-Chloro | Methyl | 17.4 | 14.7 |
| 2-Chloro | n-Propyl | 2.4 | 7.8 |
| 2-Chloro | iso-Butyl | 24.3 | 25.0 |
| 2-Chloro | n-Hexyl | 10.5 | 20.5 |
| 2-Chloro | 2-Hydroxyethyl | 29.2 | 100 |
| 2-Chloro | 3-Hydroxypropyl | 12.4 | 27.4 |
| 2-Chloro | 5-Hydroxypropyl | 15.0 | 23.7 |

| | | | |
|---|---|---|---|
| 2-Chloro | 6-Hydroxyhexyl | 50.0 | 8.7 |
| 2-Chloro | Allyl | 17.7 | 18.9 |
| 2-Chloro | 2-Acetyloxyethyl | 41.5 | 64.2 |
| 2-Chloro | 2-Phenoxyethyl | 47.0 | 73.4 |
| 2-Chloro | Ethoxyethyl | 10.7 | 20.3 |
| 2-Chloro | Cyanomethyl | 54.4 | 3.4 |
| 2-Chloro | 2-Oxopropyl | 14.7 | 20.4 |
| 2-Chloro | Ethoxycarbonylmethyl | 16.0 | 17.4 |
| 2-Chloro | 2-Hydroxypropyl | 10.4 | 15.6 |
| 2-Chloro | 2,3-Dihydroxypropyl | 15.3 | 19.2 |
| 2-Chloro | 2-Isobutoxyethyl | 13.3 | 25.4 |
| 2-Chloro | 2-(2-Hydroxyethoxy)ethyl | 10.7 | 25.0 |
| 2-Chloro | 2-(2-Ethoxyethoxy)ethyl | 5.4 | 10.2 |
| 2-Chloro | 2-Oxo-3-(ethoxycarbonyl)-propyl | 20.3 | 12.7 |
| 2-Fluoro | Ethyl | 28.5 | 33.3 |
| 2-Fluoro | 2-Hydroxyethyl | 28.5 | 14.0 |
| 3-Chloro | Ethyl | 20.1 | 62.5 |
| 3-Chloro | H | 19.3 | 20.7 |
| 3-Chloro | 2-Hydroxyethyl | 18.6 | 55.5 |
| 4-Chloro | Ethyl | 14.7 | 12.4 |
| 4-Chloro | 2-Hydroxyethyl | 17.0 | 21.3 |
| 2,4-Dichloro | Ethyl | 26.2 | 55.6 |
| 2,4-Dichloro | H | 23.4 | 53.6 |
| 2,4-Dichloro | Methyl | 17.5 | 45.5 |
| 3,4-Dichloro | H | 22.7 | 24.2 |
| 2-Methyl | Ethyl | 10.4 | 19.8 |
| 2-Methyl | 2-Hydroxyethyl | 10.8 | 5.2 |
| 4-Methyl | Ethyl | 22.4 | 30.3 |
| 4-Methyl | H | 23.5 | 26.7 |
| 4-Methyl | 2-Hydroxyethyl | 35.0 | 27.3 |

| | | | |
|---|---|---|---|
| 2,4-Dimethyl | Ethyl | 25.7 | 78.6 |
| 2,4-Dimethyl | H | 24.2 | 22.8 |
| 2,4-Dimethyl | 2-Hydroxyethyl | 19.4 | 15.4 |
| 3,4-Dimethyl | Ethyl | 17.3 | 10.4 |
| 3,4-Dimethyl | 2-Hydroxyethyl | 20.0 | 10.8 |
| 4-Isopropyl | Ethyl | 33.4 | 44.0 |
| 4-n-Octyl | Ethyl | 21.5 | 23.8 |
| 4-n-Octyl | H | 22.0 | 33.7 |
| 4-Methoxy | Ethyl | 3.2 | 20.3 |
| 4-Methoxy | 2-Hydroxyethyl | 12.7 | 25.4 |
| 2,4-Dimethoxy | Ethyl | 14.0 | 95.0 |
| 2,4-Dimethoxy | 2-Hydroxyethyl | 18.4 | 63.3 |
| 3,4-Dimethoxy | Ethyl | 16.4 | 20.4 |
| 3,4-Dimethoxy | H | 13.2 | 21.7 |
| 3,4-Dimethoxy | 2-Hydroxyethyl | 19.4 | 18.3 |
| 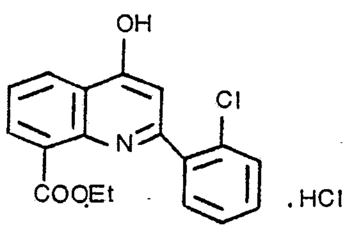 | | 29.7 | 70.5 |

Antiinflammatory action of the present invention compounds were evaluated by measuring the inhibition against carrageenin edema as explained below.

Carrageenin edema at hind foot of rats:

Experimental method.

SD-strain rats of body weight of around 150 grams were used, each group consisting of five rats. A solution (0.1 ml) of 0.5% carrageenin dissolved in physiological saline water was subcutaneously injected into right hind foot pat of the rats and, one hour prior to the injection of carrageenin, 200 mg/kg of test compound was given per os. Foot volumes of the rats before the carrageenin treatment and those of three hours after the treatment were measured, and the differences were compared with those of the control group to adopt as a target of the effect of the compounds.

Results of the representative compounds are as follows:

| Example Numbers | % Inhibition |
|---|---|
| 20 | 10.6 |
| 4 | 14.2 |
| 41 | 18.9 |
| 42 | 11.8 |
| 22 | 14.8 |

| Example Numbers | % Inhibition |
|---|---|
| 38 | 12.8 |
| 28 | 15.9 |
| 70 | 17.1 |
| 64 | 10.1 |
| 39 | 30.0 |
| 83 | 22.5 |
| 86 | 29.5 |
| 76 | 14.5 |
| 70 | 15.0 |
| 99 | 36.8 |
| 80 | 14.7 |
| 109 | 30.7 |
| 124 | 14.5 |
| 120 | 29.4 |
| 112 | 10.0 |
| 130 | 18.3 |
| 125 | 17.2 |
| 106 | 50.9 |
| 102 | 56.5 |
| 108 | 51.0 |
| 118 | 27.9 |
| 136 | 26.9 |
| 110 | 19.2 |
| 133 | 10.0 |
| 131 | 29.9 |
| 104 | 53.7 |
| 98 | 16.1 |
| 1 | 34.0 |
| (Control: Acetylsalicylic Acid | 31.6) |

Action against Platelet Coagulation:

Male rabbits with body weights of 3 kg or around were used. Blood was taken from their carotid artery, and immediately thereafter, 1/10 volume (to the blood) of 3.8% aqueous solution of sodium citrate was added thereto. The mixture was centrifuged at room temperature (400 g; 10 minutes) to give platelet rich plasma (abbreviated as PRP: containing $5 \times 10^5$ platelets per $mm^3$). The PRP was then placed into a cell for platelet coagulation meter (Niko Bioscience), stirred, 5 microliters of test compound solution in dimethyl sulfoxide was added and, one minute thereafter, 5 micromolar of ADP, 10 micrograms/ml of collagen or 25 microliters of 150 μM arachidoic acid (AA) was added thereto to observe changes of extinctions.

From the maximum coagulation value at the time of test compound addition against that at the time of solvent addition, inhibition was calculated and the effect of the compounds against the platelet coagulation was considered.

Effect of the present invention compounds and indomethacin on platelet aggregation induced by ADP, collagen and AA in platelet rich plasma of rabbits

| Name of Compound | Final Concentration (M) | Inhibition (%) ADP (5 μM) | Collagen (10 μg/ml) | AA (150 μM) |
|---|---|---|---|---|
| Example 83 | $10^{-5}$ | — | 14.0 | 3.3 |
| | $10^{-4}$ | 18.5 | 100.0 | 100 |
| Indo-metha-cin | $10^{-6}$ | — | 19.6 | 1.6 |
| | $10^{-5}$ | — | 100.0 | 100.0 |
| | $10^{-4}$ | 2.6 | 100.0 | 100.0 |

Acute toxicity is observed for two weeks after oral administration of 4000 mg/kg to male mice. Four mice (denominator) are used in one group and the dead numbers are given in numerator. Acute toxicities are all weak. Representative examples are given in Table 2.

TABLE 2

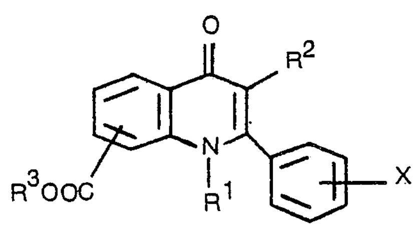

| Example No. | $R^1$ | $R^2$ | $R^3$ | X | Lethal Ratio |
|---|---|---|---|---|---|
| 20 | n-$C_6H_{13}$ | H | (6-position) H | H | 1/4 |
| 28 | H | H | (6-position) $CH_3$ | 2'—I | 0/4 |
| 42 | H | H | (6-position) H | 2'—$CH_3$ | 0/4 |
| 56 | H | H | (6-position) $CH_3$ | 2'—$OCH_3$ | 1/4 |
| 83 | H | H | (8-position) $C_2H_5$ | 2'—Cl | 0/4 |
| 84 | H | H | (8-position) Na | 2'—Cl | 0/4 |
| 85 | H | H | (8-position) H | 2'—Cl | 0/4 |
| 89 | H | H | (8-position) $C_2H_5$ | 2',4'—$Cl_2$ | 0/4 |
| 94 | H | H | (8-position) $C_2H_5$ | 2',4'—$(CH_3)_2$ | 1/4 |
| 100 | H | H | (8-position) $C_2H_5$ | 2'—Cl | 0/4 |

Lethal ratios for all other compounds are 0/4 at doses of 2000 mg/kg. It is therefore apparent that they are all safe compounds.

The mode of administration of the present invention compounds is generally as follows. Thus, in oral administration, once to thrice daily and 1 to 1000 mg each; in rectal administration, once to thrice daily and 1 to 500 mg each; in inhalation, twice to thrice to bronchi, 0.1 to 100 mg each; in intravenous administration, thrice to four times daily and 0.1 to 50 mg each; in nasal administration, twice to thrice daily and 0.1 to 100 mg each; as eye drop, thrice to four times daily and 0.1 to 50 mg each; as ointment, twice to thrice daily and 1 to 100 mg each.

The present invention compounds can be applied to preparatory compositions by the following methods. Such preparatory compositions can be practically used using, if desired, suitable pharmaceutical carrier or bulking agent, by conventional route. It is desired that those compositions are offered as a form adequate for being absorbed from stomach and intestinal canals. Still it is all right to administer them by non-oral route.

Examples of unit dose administration form are tablets, powders, fine particles, pills, granules and capsules. They may contain conventional vehicles or diluents such as binders (such as sirup, gum arabicum, gelatin, sorbitol, tragacanth, polyvinyl pyrrolidone, polyvinyl alcohol, hydroxypropyl methyl cellulose, hydroxypropyl cellulose, methyl cellulose, carboxymethyl cellulose sodium, and the like), diluents (such as lactose, sucrose, corn starch, calcium phosphate, sorbitol, crystalline cellulose and the like), lubricants (such as magnesium stearate, talc, polyethyleneglycol, silica and the like), disintegrating agents (such as potato starch, hydroxypropyl cellulose with lower degree of substitution, microcrystalline cellulose and the like), acceptable wetting agents (such as sodium laurylsulfate and the like), and the like. Tablets may be subjected to coating by a known method.

Liquidal preparations may be oil suspensions, solutions, sirups, elixiliers and the like and may be dried product which is redissolved in suitable vehicles such as water before use. Those liquidal preparations may contain conventional additives. They are, for example, suspending agents such as methylcellulose, carboxymethylcellulose sodium, hydroxypropyl cellulose, hydroxypropyl methyl cellulose, polyvinyl pyrrolidone, polyvinyl alcohol, acacia, tragacanth, gelatin, sodium alginate and the like; emulsifiers such as lecithin, sorbitan, fatty acid esters, gum arabicum, tragacanth and the like; wetting agents such as polyoxyethylene sorbitan fatty acid esters, polyoxyethylene fatty acid esters, polyoxyethylene derivatives of hydrogenated castor oil and the like; nonaqueous vehicles such as sesami oil, soybean oil and other plant oils, propylene glycol, polyethylene glycol, ethyl alcohol and the like; antiseptics such as methyl p-hydroxybenzoate, propyl p-hydroxybenzoate, sorbic acid and the like; sweetening agents such as simple sirup, sucrose, sorbitol, mannitol.

As to bases for rectal administration, fatty or oily bases such as cacao fat, triglyceride (Widepsol — Registered Trademark), and the like or water-soluble bases such as polyethylene glycol and the like can be used. The so-called rectal capsules in which the active constituent is suspended in vegetable oil and made into gelatin capsules are also used.

Those preparations may be made into long acting preparations by known methods and forms or may be made into microcapsules.

It is desired that about 0.1 to 99% (or 0.5 to 90% in general) of one or more of the present invention effective compounds is contained in total compositions in the preparations.

Examples of manufacturing pharmaceutical preparations containing the present invention compounds as main constituents are given as hereunder. In the examples, amounts (usually in "mg") are to be understood that the constituent are taken in such a ratio by weight.

Examples for Manufacturing Pharmaceutical Preparations:

(1) Capsules containing ethyl 2-(2-chlorophenyl)-4-quinolone-8-carboxylate (a compound of Example 83) as a main constituent.

A compound of Example 83 and bulking agents are uniformly mixed as per the following ratio and filled in hard gelatine capsules to afford desired preparations.

| | |
|---|---|
| Compound of Example 83 | 50 mg |
| Lactose | 104 mg |
| Potato starch | 40 mg |
| Talc | 5 mg |
| Magnesium stearate | 1 mg |
| added to make | 200 mg per capsule |

(2) Tablets containing methyl 2-(4-n-pentylphenyl)-4-quinolone-6-carboxylate (a compound of Example 50) as a main constituent.

Pulverized compound of Example 50 (100 mg), 100 mg of lactose, 75 mg of crystalline cellulose and 40 mg of potato starch are mixed, kneaded after addition of binder solution prepared from 10 mg of polyvinyl alcohol, passed through a sieve of 16 mesh to afford grains, then dried, and size of the grains are made

uniform by passing through a sieve of 16 mesh. The resulting granules are mixed with 3 mg of magnesium stearate and 7 mg of talc and compressed into tablets. The resulting tablets are, if desired, coated using the conventional coating material or sugar and the like.

Manufacture of the present invention compounds are then illustrated as hereunder.

Reference Example 1

Ethyl o-chlorobenzoylacetate,

Sodium hydride (8.7 grams) and 21.4 grams of diethyl carbonate are suspended in 100 ml of tetrahydrofuran, 14 grams of o-chloroacetophenone is gradually dropped thereinto by keeping the inner temperature at 40 to 50°C, then small amount of ethanol is added thereto and heated to reflux for four hours. After cooled, 20 ml of ethanol is added thereto, the mixture is poured into ice water, and extracted with ether. Ether extracts are combined, washed with water, dried with anhydrous magnesium sulfate, and ether is evaporated therefrom. The resulting oil is purified by distillation in vacuo to give 8.8 grams of colorless, oily product, b.p. 120 to 125° C/l mmg Hg.

Reference Example 2

Ethyl p-isopropylbenzoylacetate.

Sodium hydride (2.4 grams) and 4.5 grams of dimethyl carbonate are suspended in 150 ml of ether, 8.1 grams of p-isopropylacetophenone is gradually dropped thereinto under heating to reflux, then small amount of ethanol is added thereto, heated to reflux for one hour, cooled, and the reaction is made stopped by addition of 20 ml of ethanol. The mixture is poured into ice water and extracted with ether. The ether extracts are combined, washed with water, dried with anhydrous magnesium sulfate and ether is evaporated therefrom. The resulting oil is purified by distilling in vacuo to give 6.5 grams of colorless oily product, b.p. 80° C/5 mmHG.

Reference Example 3

Ethyl 2-furoylacetate.

Sodium hydride (4.4 grams) and 10.7 grams of diethyl carbonate are suspended in 60 ml of tetrahydrofuran and 10 grams of 2-acetylfuran is gradually added thereto by keeping the inner temperature at 40 to 50°C. Small amount of ethanol is added thereto, heated to reflux for three hours, and the reaction is made stopped by addition of 20 ml of ethanol. The mixture is poured into ice water, extracted with ether, the ether extracts are combined, washed with water, dried with anhydrous magnesium sulfate, and ether is evaporated therefrom. The resulting oil is purified by distilling in vacuo to give 10 grams of pale yellow oily product, b.p. 90° C/3 mm Hg.

Reference Example 4

Ethyl m-trifluoromethylbenzolacetate

Cyclohexyl isopropylamine (16.4 grams) is dissolved in 100 ml of tetrahydrofuran and the solution is cooled at −78°C. In a nitrogen atmosphere, 40 ml of about 15% n-butyl lithium solution in hexane is added thereto. The mixture temperature is raised up to −40°C during thirty minutes. Then it is cooled to −78°C again and 4.5 grams of ethyl acetate is added thereto during five minutes. Then, after ten minutes thereafter, a solution of 12.1 grams of m-trifluoromethylbenzoic acid chloride in tetrahydrofuran is dropped thereinto gradually. The mixture is stirred at −78°C for one hour then raised to 0°C during two hours. To this is added 20 ml of 20% hydrochloric acid solution, the mixture is poured into ice water, and extracted with ether. The extract is washed with saturated aqueous solution of sodium bicarbonate, dried with anhydrous magnesium sulfate, ether is evaporated therefrom, and the resulting oil is purified by distilling in vacuo to give 10.7 grams of pale yellow oil, b.p. 125 to 130°C/5 mmHg.

Reference Example 5

Ethyl o-iodobenzoylacetate.

Cyclohexyl isopropylamine (10.6 grams) is dissolved in 80 ml of tetrahydrofuran and the solution is cooled to −78°C. In a nitrogen stream, a solution of n-butyl lithium (about 15%) in hexane solution (26 ml) is added thereto, and the mixture temperature is raised to −40°C. Then it is cooled to −78°C once again, 3,3 grams of ethyl acetate is gradually added thereto and, ten minutes thereafter, a solution of 10 grams of o-iodobenzoyl chloride in tetrahydrofuran is dropped thereinto gradually. The mixture is stirred at −78°C for one hour, then gradually warmed up to 0°C within two to three hours, 20 ml of 20% hydrochloric acid solution is added thereto, the mixture is poured into ice water, and extracted with ether. The extract is washed with saturated aqueous solution of sodium bicarbonate, dried with anhydrous magnesium sulfate, ether is evaporated therefrom, and the resulting oil is purified by distilling in vacuo to give 8.5 grams of pale yellow oil, b.p. 142°C/3 mmHg.

Reference Example 6

Ethyl alpha-allylbenzoylacetate

Ethyl benzoylacetate (6 grams) is dissolved in 40 ml of dimethyl formamide and 1.5 grams of sodium hydride is added thereto with ice cooling and stirring. Ten minutes thereafter, 4 grams of allyl bromide is

added thereto, the mixture is stirred at room temperature for one hour, poured into water, the mixture is neutralized with acetic acid, and extracted with ether. The ether extract is washed with water, dried with anhydrous magnesium sulfate, and ether is evaporated therefrom to give 6.1 grams of colorless oily product.

Example 1

Methyl 2-phenyl-4-quinolone-6-carboxylate.

Methyl p-aminobenzoate (3.0 grams) and 4.1 grams of ethyl benzoylacetate are dissolved in 100 ml of chloroform and the solution is subjected to azeotropic dehydration for two days after addition of 0.5 gram of p-toluenesulfonic acid. Chloroform is evaporated therefrom, n-hexane is added to the residue. The extract with n-hexane is evaporated, 30 ml of diphenyl ether is added to the residue, the mixture is heated at 255°C for twenty minutes, cooled, crystals separated out therefrom are collected by filtration, and washed with n-hexane and dried to give 4.4 grams of methyl 2-phenyl-4-quinolone-6-carboxylate, needles, melting at higher than 300°C.

Elementary analysis calculated as $C_{17}H_{13}NO_3$: C 73.11, H 4.69, N 5.02; Found: C 73.33, H 4.45, N 5.05.

Example 2

2-Phenyl-4-quinolone-6-carboxylic acid.

Methyl 2-phenyl-4-quinolone-6-carboxylate (3.8 grams) obtained in Example 1 is refluxed for three hours in a mixture of 100 ml of methanol, 3 grams of sodium hydroxide and 15 ml of water. The mixture is evaporated to dryness in vacuo, 100 ml of water is added to the residue, filtered, acidified with 10% hydrochloric acid solution, and the crystals separated out therefrom are collected by filtration. These are recrystallized from dimethyl formamide to give 2.8 grams of 2-phenyl-4-quinolone-6-carboxylic acid, colorless powder, melting at above 300°C.

Elementary analysis calculated for $C_{16}H_{11}NO_3$: C 72.44, H 4.18, N 5.28; Found: C 72.44, H 3.89, N 5.22.

Example 3

Sodium 2-phenyl-4-quinolone-6-carboxylate.

2-Phenyl-4-quinolone-6-carboxylic acid (2.5 grams) obtained in Example 2 is dissolved, together with 0.8 gram of sodium hydroxide, in 50 ml of water with heating, the solution is concentrated, diluted with ethanol, and the crystals separated out therefrom are collected by filtration. These are dried by heating to give 2.2 grams of sodium salt of 2-phenyl-4-quinolone-6-carboxylic acid, colorless powder, melting at above 300°C.

Elementary analysis calculated for $C_{16}H_{10}NO_3Na$: C 66.89, H 3.52, N 4.88; Found: C 66.67, H 3.74, N 4.77.

Example 4

Ethyl 2-phenyl-4-quinolone-6-carboxylate

2-Phenyl-4-quinolone-6-carboxylic acid (1.3 grams) obtained in Example 2 is dissolved in 80 ml of ethanol and 0.5 ml of concentrated sulfuric acid, the solution is stirred for three days at 80°C, water is added thereto, crystals separated out therefrom are collected by filtration, these are washed with water and dried to give 1.4 grams of ethyl 2-phenyl-4-quinolone-6-carboxylate, colorless powder, melting at above 300°C.

Elementary analysis calculated for $C_{18}H_{15}NO_3$: C 73.70, H 5.15, N 4.78; Found: C 73.52, H 5.30, N 4.76.

Example 5

Isopropyl 2-phenyl-4-quinolone-6-carboxylate.

Metal sodium (0.33 gram) is dissolved in 40 ml of anhydrous isopropyl alcohol and the solution is heated to reflux for one hour with 2 grams of 2-phenyl-4-quinolone-6-carboxylic acid obtained in Example 2. After cooled, the solution is mixed with ice water, acidified with acetic acid, the crystals separated out therefrom are collected by filtration, the crystals are washed with water, washed with methanol, and dried to give 0.6 gram of isopropyl 2-phenyl-4-quinolone-6-carboxylate, colorless powder, melting at above 300°C.

Elementary analysis calculated for $C_{19}H_{17}NO_3$: C 74.25, H 5.58, N 4.56; Found: C 73.99, H 54.6, N 4.31.

Example 6

Phenethyl 2-phenyl-4-quinolone-6-carboxylate.

2-Phenyl-4-quinolone-6-carboxylate (1.5 grams) obtained in Example 2 is stirred for fifteen hours at 110°C with 50 ml phenethyl alcohol and 1 ml of concentrated sulfuric acid. After cooled, 1.5 liters of ether is added to the mixture, the whole is cooled with ice, oil separated out therefrom is taken out, and it is dissolved in 5 ml of ethanol. Then 3 ml of water is added thereto, crystals separated out therefrom are collected by filtration, washed with 5% sodium bicarbonate solution, then with water, and finally with methanol, and dried to give 0.9 gram of phenethyl 2-phenyl-4-quinolone-6-carboxylate, colorless powder, melting point 282 to 284°C.

Elementary analysis calculated for $C_{24}H_{19}NO_3$: C 78.03, H 5.18, N 3.79; Found: C 77.80, H 5.27, N 3.64.

Example 7

Methyl 1-methyl-2-phenyl-4-quinolone-6-carboxylate.

A mixture of 2 grams of methyl 2-phenyl-4-quinolone-6-carboxylate (obtained in Example 1), 16 ml of methyl iodide, 2 grams of potassium carbonate and 50 ml of dimethyl formamide is stirred for four hours at 60°C. After cooled, the mixture is poured into water and extracted with ethyl acetate. From the extract is evaporated ethyl acetate, the residue is purified by silica chromatography, and 1.8 grams of methyl 1-methyl-2-phenyl-4-quinolone-6-carboxylate, colorless powder, melting point 174 to 176°C, is obtained.

Elementary analysis calculated for $C_{18}H_{15}NO_3$: C 73.70, H 5.15, N 4.78; Found: C 74.84, H 4.96, N 4.71.

Example 8

1-Methyl-2-phenyl-4-quinolone-6-carboxylic acid.

To 0.9 gram of methyl 1-methyl-2-phenyl-4-quinolone-6-carboxylate obtained in Example 7 are added to 0.5 gram of sodium hydroxide and 40 ml of water and the mixture is stirred at 60°C for two hours. This is acidified with acetic acid, crystals separated out therefrom are collected by filtration, and dried to give 0.5 gram of 1-methyl-2-phenyl-4-quinolone-6-carboxylic acid, colorless powder, melting at above 300°C.

Elementary analysis calculated for $C_{17}H_{13}NO_3$: C 73.11, H 4.69, N 5.02; Found: C 73.18, H 4.44, N 4.85.

Example 9

Methyl 3-methyl-2-phenyl-4-quinolone-6-carboxylate.

Ethyl alpha-methylbenzoylacetate (5 grams) and 3.67 grams of methyl p-aminobenzoate are heated for three days in 100 ml of chloroform in the presence of 0.7 gram of p-toluenesulfonic acid to dehydrate. *p*-Toluenesulfonic acid is removed therefrom, the filtrate is concentrated, and purified by silica gel column chromatography to give oil. The resulting oily product is heated to reflux in 70 ml of diphenyl ether, cooled, and crystals separated therefrom are collected by filtration and recrystallized from a mixture of dimethyl formamide and water to give 2 grams of methyl 3-methyl-2-phenyl-4-quinolone-6-carboxylate, colorless crystals, melting point above 300°C.

Elementary analysis calculated for $C_{18}H_{15}NO_3$: C 73.70, H 5.15, N 4.78; Found: C 73.84, H 5.03, N 4.86.

Compounds of Example 10 to Example 79 are synthesized by the use of the method given in Example 1 to Example 9. They are given in the following tables.

In case Z is phenyl

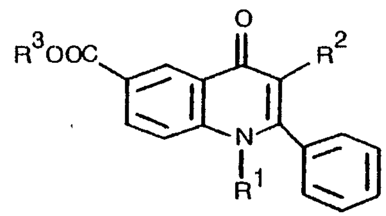

| Example No. | $R^1$ | $R^2$ | $R^3$ | Appearance | M.p. | Elementary Analysis |
|---|---|---|---|---|---|---|
| 10 | H | Me | H | Colourless Crystals | >300°C | $C_{17}H_{17}NO_3$<br>Calcd. (%) C 73.11 H 4.69 N 5.02<br>Found. (%) C 73.05 H 4.47 N 4.98 |
| 11 | H | $CH_2CH{=}CH_2$ | Me | " | 235—236°C | $C_{20}H_{17}NO_3$<br>Calcd. (%) C 75.22 H 5.37 N 4.39<br>Found. (%) C 75.13 H 5.59 N 4.44 |
| 12 | H | $CH_2CH{=}CH_2$ | H | " | >—300°C | $C_{19}H_{15}NO_3$<br>Calcd. (%) C 74.74 H 4.95 N 4.59<br>Found. (%) C 74.79 H 1.72 N 4.55 |
| 13 | Et | H | Me | Colorless Powder | 152—155°C | $C_{17}H_{17}NO_3$<br>Calcd. (%) C 74.25 H 5.58 N 4.56<br>Found. (%) C 74.42 H 5.49 N 4.60 |
| 14 | Et | H | H | Colorless Crystals | >300°C | $C_{18}H_{15}NO_3$<br>Calcd. (%) C 73.70 H 5.15 N 4.78<br>Found. (%) C 73.96 H 4.87 N 4.84 |
| 15 | n-$C_3H_7$ | H | Me | " | 133—136°C | $C_{20}H_{17}NO_3$<br>Calcd. (%) C 74.74 H 5.96 N 4.36<br>Found. (%) C 74.65 H 5.93 N 4.24 |
| 16 | n-$C_3H_2$ | H | H | " | 223—225°C | $C_{19}H_{17}NO_3$<br>Calcd. (%) C 74.25 H 5.58 N 4.56<br>Found. (%) C 74.22 H 5.62 N 4.64 |
| 17 | $CH_2CH{=}CH_2$ | H | Me | " | 147—149°C | $C_{20}H_{17}NO_3$<br>Calcd. (%) C 75.22 H 5.37 N 4.39<br>Found. (%) C 75.05 H 5.31 N 4.37 |

| Example No. | $R^1$ | $R^2$ | $R^3$ | Appearance | M.p. | Elementary Analysis |
|---|---|---|---|---|---|---|
| 18 | $CH_2CH=CH_2$ | H | H | Colorless Crystals | 211—213°C | $C_{19}H_{15}NO_3$<br>Calcd. (%) C 74.74 H 4.95 N 4.59<br>Found. (%) C 74.82 H 4.79 N 4.56 |
| 19 | n-$C_6H_{13}$ | H | Me | " | 105—107°C | $C_{23}H_{25}NO_3$<br>Calcd. (%) C 76.00 H 6.93 N 3.85<br>Found. (%) C 76.05 H 7.13 N 3.95 |
| 20 | n-$C_6H_{13}$ | H | H | " | 196—198°C | $C_{22}H_{23}NO_3$<br>Calcd. (%) C 75.62 H 6.63 N 4.01<br>Found. (%) C 75.35 H 6.67 N 4.04 |

In case where Z is substituted phenyl

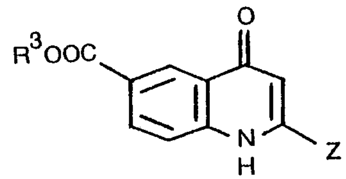

| Example No. | Substituent(s) on phenyl | $R^3$ | Appearance | M.p. | Elementary Analysis |
|---|---|---|---|---|---|
| 21 | 2'-Cl | Me | Colorless needles | >300°C | $C_{17}H_{16}ClNO_3$<br>Calcd. (%) C 65.07 H 3.86 N 4.47<br>Found. (%) C 65.30 H 3.68 N 4.26 |
| 22 | 2'-Cl | H | Colorless powder | >300°C | $C_{16}H_{10}ClNO_3$<br>Calcd. (%) C 64.12 H 3.36 N 4.67<br>Found. (%) C 63.88 H 3.28 N 4.69 |
| 23 | 2'-Cl | Na | Colorless powder | >300°C | $C_{16}H_9ClNO_3Na$<br>Calcd. (%) C 52.34 H 3.81 N 3.81<br>Found. (%) C 52.62 H 3.53 N 3.73 |
| 24 | 2'-Br | Me | Pale yellow needles | 294—295°C | $C_{17}H_{12}BrNO_3$<br>Calcd. (%) C 57.00 H 3.38 N 3.91<br>Found. (%) C 57.13 H 3.20 N 3.82 |
| 25 | 2'-Br | H | Colorless powder | >300°C | $C_{16}H_{10}BrNO_3$<br>Calcd. (%) C 55.84 H 2.93 N 4.07<br>Found. (%) C 55.89 H 2.77 N 3.90 |
| 26 | 2'-F | Me | Colorless crystals | >300°C | $C_{17}H_{16}FNO_3$<br>Calcd. (%) C 68.68 H 4.09 N 4.71<br>Found. (%) C 68.69 H 3.81 N 4.67 |
| 27 | 2'-F | H | Colorless powder | >300°C | $C_{16}H_{10}FNO_3$<br>Calcd. (%) C 67.84 H 3.59 N 4.94<br>Found. (%) C 67.71 H 3.29 N 4.87 |
| 28 | 2'-I | Me | Pale yellow needles | >300°C | $C_{17}H_{12}INO_3$<br>Calcd. (%) C 50.39 H 2.99 N 3.46<br>Found. (%) C 50.45 H 3.01 N 3.24 |

| Example No. | Substituent(s) on phenyl | $R^3$ | Appearance | M.p. | Elementary Analysis |
|---|---|---|---|---|---|
| 29 | 3'-Cl | Me | Colorless needles | >300°C | $C_{17}H_{12}ClNO_3$<br>Calcd. (%) C 65.07 H 3.86 N 4.47<br>Found. (%) C 65.26 H 3.62 N 4.42 |
| 30 | 3'-Cl | H | Colorless powder | >300°C | $C_{16}H_{10}ClNO_3$<br>Calcd. (%) C 64.12 H 3.36 N 4.67<br>Found. (%) C 63.96 H 3.19 N 4.67 |
| 31 | 3'-Cl | Na | Colorless powder | >300°C | $C_{16}H_9ClNO_3Na$<br>Calcd. (%) C 53.67 H 3.63 N 3.91<br>Found. (%) C 53.44 H 3.41 N 3.82 |
| 32 | 4'-Cl | Me | Pale blue needles | >300°C | $C_{17}H_{12}ClNO_3$<br>Calcd. (%) C 65.07 H 3.86 N 4.47<br>Found. (%) C 65.29 H 3.64 N 4.34 |
| 33 | 4'-Cl | H | Colorless powder | >300°C | $C_{16}H_{10}ClNO_3$<br>Calcd. (%) C 64.12 H 3.36 N 4.67<br>Found. (%) C 63.91 H 3.10 N 4.49 |
| 34 | 2',4'-$Cl_2$ | Me | Colorless crystals | >300°C | $C_{17}H_{11}Cl_2N_3$<br>Calcd. (%) C 58.64 H 3.18 N 4.02<br>Found. (%) C 58.91 H 2.95 N 3.88 |
| 35 | 2',4'-$Cl_2$ | H | Colorless powder | >300°C | $C_{16}H_9Cl_2NO_3$<br>Calcd. (%) C 57.51 H 2.71 N 4.19<br>Found. (%) C 57.68 H 2.47 N 3.98 |
| 36 | 2',4'-$Cl_2$ | Na | Colorless powder | >300°C | $C_{16}H_8Cl_2NO_3Na$<br>Calcd. (%) C 53.67 H 3.63 N 3.91<br>Found. (%) C 53.44 H 3.41 N 3.82 |
| 37 | 3',4'-$Cl_2$ | Me | Colorless needles | >300°C | $C_{17}H_{11}Cl_2NO_3$<br>Calcd. (%) C 58.64 H 3.18 N 4.02<br>Found. (%) C 58.90 H 2.96 N 3.85 |
| 38 | 3',4'-$Cl_2$ | H | Colorless powder | >300°C | $C_{16}H_9Cl_2NO_3$<br>Calcd. (%) C 57.46 H 2.69 N 4.19<br>Found. (%) C 57.51 H 2.45 N 4.09 |

| Example No. | Substituent(s) on phenyl | $R^3$ | Appearance | M.p. | Elementary Analysis |
|---|---|---|---|---|---|
| 39 | 2′,5′-$Cl_2$ | Me | Colorless powder | >300°C | $C_{17}H_{17}Cl_2NO_3$<br>Calcd. (%) C 58.64 H 3.18 N 4.02<br>Found. (%) C 58.88 H 3.15 N 3.94 |
| 40 | 2′,5′-$Cl_2$ | H | ″ | >300°C | $C_{16}H_9Cl_2NO_3$<br>Calcd. (%) C 57.46 H 2.69 N 4.19<br>Found. (%) C 57.40 H 2.93 N 4.37 |
| 41 | 2′-Me | Me | ″ | >300°C | $C_{18}H_{15}NO_3$<br>Calcd. (%) C 73.70 H 5.15 N 4.78<br>Found. (%) C 73.67 H 5.07 N 4.99 |
| 42 | 2′-Me | H | ″ | >300°C | $C_{17}H_{13}NO_3$<br>Calcd. (%) C 73.11 H 4.69 N 5.02<br>Found. (%) C 73.23 H 4.60 N 5.18 |
| 43 | 2′-Me | Na | ″ | >300°C | $C_{17}H_{12}NO_3Na$<br>Calcd. (%) C 62.15 H 4.56 N 4.26<br>Found. (%) C 61.98 H 4.61 N 4.12 |
| 44 | 2′-Et | Me | ″ | 249—251°C | $C_{19}H_{17}NO_3$<br>Calcd. (%) C 74.25 H 5.58 N 4.56<br>Found. (%) C 74.23 H 5.30 N 4.55 |
| 45 | 2′-Et | H | ″ | >300°C | $C_{18}H_{15}NO_3$<br>Calcd. (%) C 73.70 H 5.15 N 4.78<br>Found. (%) C 73.69 H 5.19 N 4.73 |
| 46 | 4′-Me | Me | Colorless crystals | >300°C | $C_{13}H_{15}NO_3$<br>Calcd. (%) C 73.70 H 5.15 N 4.78<br>Found. (%) C 73.90 H 5.05 N 4.75 |
| 47 | 4′-Me | H | Colorless powder | >300°C | $C_{17}H_{13}NO_3$<br>Calcd. (%) C 73.11 H 4.69 N 5.02<br>Found. (%) C 73.30 H 4.61 N 4.96 |
| 48 | 4′-iso$C_3H_7$ | Me | ″ | >300°C | $C_{20}H_{19}NO_3$<br>Calcd. (%) C 74.74 H 5.96 N 4.36<br>Found. (%) C 75.00 H 5.89 N 4.40 |

| Example No. | Substituent(s) on phenyl | $R^3$ | Appearance | M.p. | Elementary Analysis |
|---|---|---|---|---|---|
| 49 | 4'-iso$C_3H_7$ | H | Colorless powder | >300°C | $C_{19}H_{17}NO_3$<br>Calcd. (%) C 74.25 H 5.58 N 4.56<br>Found. (%) C 74.11 H 5.42 N 4.61 |
| 50 | 4'-n-$C_5H_{11}$ | Me | " | >300°C | $C_{22}H_{23}NO_3$<br>Calcd. (%) C 75.62 H 6.63 N 4.01<br>Found. (%) C 75.76 H 6.72 N 4.14 |
| 51 | 4'-n-$C_5H_{11}$ | H | " | >300°C | $C_{21}H_{21}NO_3$<br>Calcd. (%) C 75.20 H 6.31 N 4.18<br>Found. (%) C 75.19 H 6.32 N 4.21 |
| 52 | 3'-$CF_3$ | Me | " | >300°C | $C_{18}H_{12}F_3NO_3$<br>Calcd. (%) C 62.25 H 3.48 N 4.03<br>Found. (%) C 62.48 H 3.37 N 3.92 |
| 53 | 3'-$CF_3$ | H | " | >300°C | $C_{17}H_{10}F_3NO_3$<br>Calcd. (%) C 61.27 H 3.02 N 4.20<br>Found. (%) C 61.23 H 2.90 N 4.38 |
| 54 | 2',4'-(Me)$_2$ | Me | Colorless needles | >300°C | $C_{19}H_{17}NO_3$<br>Calcd. (%) C 74.25 H 5.58 N 4.56<br>Found. (%) C 74.50 H 5.34 N 4.40 |
| 55 | 2',4'-(Me)$_2$ | H | Colorless powder | >300°C | $C_{18}H_{15}NO_3$<br>Calcd. (%) C 73.70 H 5.15 N 4.78<br>Found. (%) C 73.57 H 4.93 N 4.81 |
| 56 | 2'-OMe | Me | " | 277—278°C | $C_{18}H_{15}NO_4$<br>Calcd. (%) C 69.89 H 4.89 N 4.53<br>Found. (%) C 70.05 H 4.76 N 4.39 |
| 57 | 2'-OMe | H | " | 290—294°C | $C_{17}H_{13}NO_5 \cdot H_2O$<br>Calcd. (%) C 65.17 H 4.79 N 4.44<br>Found. (%) C 65.11 H 4.53 N 4.34 |
| 58 | 2'-OEt | Me | Colorless needles | 244—245°C | $C_{19}H_{17}NO_4$<br>Calcd. (%) C 70.57 H 5.30 N 4.33<br>Found. (%) C 70.78 H 5.31 N 4.35 |

| Example No. | Substituent(s) on phenyl | $R^3$ | Appearance | M.p. | Elementary Analysis |
|---|---|---|---|---|---|
| 59 | 2'-OEl | H | Colorless prisms | >300°C | $C_{18}H_{15}NO_4$<br>Calcd. (%) C 69.89 H 4.89 N 4.53<br>Found. (%) C 70.02 H 4.83 N 4.55 |
| 60 | 2'-O-iso$C_3H_7$ | Me | Colorless crystals | 255—256°C | $C_{20}H_{19}NO_4$<br>Calcd. (%) C 71.20 H 5.68 N 4.15<br>Found. (%) C 71.19 H 5.66 N 4.10 |
| 61 | 2'-O-iso$C_3H_7$ | H | Colorless powder | >300°C | $C_{19}H_{17}NO_4$<br>Calcd. (%) C 70.57 H 5.30 N 4.33<br>Found. (%) C 70.70 H 5.20 N 4.28 |
| 62 | 4'-OMe | Me | Colorless needles | >300°C | $C_{18}H_{15}NO_4$<br>Calcd. (%) C 69.89 H 4.89 N 4.53<br>Found. (%) C 70.00 H 4.73 N 4.49 |
| 63 | 4'-OMe | H | Colorless needles | >300°C | $C_{17}H_{13}NO_4 \cdot \frac{1}{2}H_2O$<br>Calcd. (%) C 67.10 H 4.60 N 4.60<br>Found. (%) C 67.37 H 4.30 N 4.52 |
| 64 | 2',4'-$(OMe)_2$ | Me | Colorless crystals | 272—273°C | $C_{19}H_{17}NO_5$<br>Calcd. (%) C 67.25 H 5.05 N 4.13<br>Found. (%) C 66.94 H 5.11 N 3.94 |
| 65 | 2',4'-$(OMe)_2$ | H | Pale yellow powder | >300°C | $C_{18}H_{15}NO_5$<br>Calcd. (%) C 66.45 H 4.65 N 4.31<br>Found. (%) C 66.53 H 4.55 N 4.24 |
| 66 | 3',4'-$(OMe)_2$ | Me | Colorless crystals | >300°C | $C_{19}H_{17}NO_5$<br>Calcd. (%) C 67.25 H 5.05 N 4.13<br>Found. (%) C 67.34 H 4.55 N 4.04 |
| 67 | 3',4'-$(OMe)_2$ | H | Pale yellow powder | >300°C | $C_{18}H_{15}NO_5 \cdot H_2O$<br>Calcd. (%) C 62.97 H 4.99 N 4.08<br>Found. (%) C 62.99 H 4.71 N 4.06 |
| 68 | 4'-COOMe | Me | Pale yellow powder | >300°C | $C_{19}H_{15}NO_5$<br>Calcd. (%) C 67.65 H 4.48 N 4.15<br>Found. (%) C 67.94 H 4.23 N 3.86 |
| 69 | 4'-COOH | H | Colorless powder | >300°C | $C_{17}H_{11}NO_5$<br>Calcd. (%) C 66.02 H 3.59 N 4.53<br>Found. (%) C 65.85 H 3.60 N 4.51 |

Those compounds as listed hereinabove can, of course, be manufactured by other routes. For example, the compound of Example 57 was prepared by the following way too.

Thus, to 4 grams of 6-cyano-2-(2-methoxyphenyl)-4-quinolone were added 3 grams of potassium hydroxide and 100 ml of methanol, the mixture was heated to reflux for 4 hours, then poured over into ice water, the mixture was adjusted to pH 3 with 10% hydrochloric acid, crystals separated out therefrom were collected by filtration, and recrystallized from dimethyl formamide to give 2.7 grams of the desired 2-(2-methoxyphenyl)-4-quinolone-6-carboxylic acid, colorless crystals, melting point 290 to 294°C.

In case where Z is heterocyclic ring

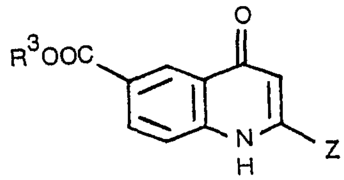

| Example No. | Z | $R_3$ | Appearance | M.p. | Elementary Analysis |
|---|---|---|---|---|---|
| 70 | 4-Pyridyl | Me | Pale brown crystals | >300°C | $C_{16}H_{12}N_2O_3$<br>Calcd. (%) C 68.56 H 4.32 N 10.00<br>Found. (%) C 68.86 H 4.06 N 9.88 |
| 71 | 4-Pyridyl | H | Pale yellow powder | >300°C | $C_{15}H_{10}N_2O_3$<br>Calcd. (%) C 67.66 H 3.79 N 10.52<br>Found. (%) C 67.76 H 3.63 N 10.33 |
| 72 | 3-Pyridyl | Me | Colorless crystals | >300°C | $C_{16}H_{12}N_2O_3$<br>Calcd. (%) C 68.56 H 4.32 N 10.00<br>Found. (%) C 68.75 H 4.13 N 9.87 |
| 73 | 3-Pyridyl | H | Colorless powder | >300°C | $C_{15}H_{10}N_2O_3$<br>Calcd. (%) C 67.66 H 3.79 N 10.52<br>Found. (%) C 67.45 H 3.62 N 10.24 |
| 74 | 2-Pyridyl | Me | Pale brown crystals | >300°C | $C_{16}H_{12}N_2O_3$<br>Calcd. (%) C 68.56 H 4.32 N 10.00<br>Found. (%) C 68.85 H 4.20 N 10.02 |
| 75 | 2-Pyridyl | H | Colorless crystals | >300°C | $C_{15}H_{10}N_2O_3$<br>Calcd. (%) C 67.66 H 3.79 N 10.52<br>Found. (%) C 67.59 H 3.77 N 10.36 |
| 76 | 2-Furyl | Me | " | >300°C | $C_{15}H_{11}NO_4$<br>Calcd. (%) C 66.91 H 4.12 N 5.20<br>Found. (%) C 67.13 H 3.93 N 5.13 |
| 77 | 2-Furyl | H | " | >300°C | $C_{14}H_9NO_4$<br>Calcd. (%) C 65.88 H 3.55 N 5.49<br>Found. (%) C 65.75 H 3.42 N 5.40 |
| 78 | 2-thienyl | Me | Colorless Crystals | >300°C | $C_{15}H_{11}NO_3S$<br>Calcd. (%) C 63.16 H 3.89 N 4.91<br>Found. (%) C 63.25 H 3.68 N 4.82 |
| 79 | 2-thienyl | H | " | >300°C | $C_{14}H_9NO_3S$<br>Calcd. (%) C 61.99 H 3.34 N 5.16<br>Found. (%) C 61.95 H 3.15 N 5.06 |

Example 80

Ethyl 2-phenyl-4-quinolone-8-carboxylate

A mixture of 7 grams of ethyl benzoylacetate, 6 grams of ethyl anthranilate and 0.5 gram of p-toluenesulfonic acid is heated to dehydrate in 300 ml of benzene for three days. Benzene is evaporated therefrom, the resulting oil is heated at 250 to 280°C for 0.5 hour in 20 ml of diphenyl ether. After cooled, 250 ml of hexane is added thereto, crystals separated out therefrom are collected by filtration and recrystallized from methanol to give 4.2 grams of ethyl 2-phenyl-4-quinolone-8-carboxylate, pale brown needles, melting point 218 to 219°C.

Elementary analysis calculated for $C_{17}H_{13}NO_3$: C 73.11, H 4.69, N 5.02; Found: C 73.04, H 4.85, N 4.81.

Compounds of examples 81 to 99 are given in the following table.

In case where Z is substituted phenyl

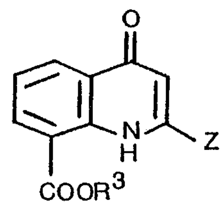

| Example No. | Substituent(s) on phenyl | $R^3$ | Appearance | M.p. | Elementary Analysis |
|---|---|---|---|---|---|
| 81 | H | H | Colorless crystals | 295—296°C | $C_{16}H_{11}NO_3$<br>Calcd. (%) C 72.44 H 4.18 N 5.28<br>Found. (%) C 72.28 H 3.89 N 5.01 |
| 82 | H | Na | Colorless powder | >300°C | $C_{16}H_{10}NO_30Na \cdot H_2O$<br>Calcd. (%) C 62.95 H 3.96 N 4.59<br>Found. (%) C 62.68 H 3.99 N 4.33 |
| 83 | 2′-Cl | Et | Colorless needles | 147—148°C | $C_{18}H_{14}ClNO_3$<br>Calcd. (%) C 65.96 H 4.31 N 4.27<br>Found. (%) C 66.24 H 4.16 N 4.43 |
| 84 | 2′-Cl | Na | Colorless prisms | 262—268°C | $C_{16}H_9ClNO_3Na$<br>Calcd. (%) C 59.73 H 2.81 N 4.35<br>Found. (%) C 59.55 H 3.00 N 4.23 |
| 85 | 2′-Cl | H | Colorless powder | >300°C | $C_{16}H_{10}ClNO_3$<br>Calcd. (%) C 64.12 H 3.36 N 4.67<br>Found. (%) C 64.00 H 3.48 N 4.90 |
| 86 | 3′-Cl | Et | Pale yellow needles | 177—178°C | $C_{18}H_{14}ClNO_3$<br>Calcd. (%) C 65.96 H 4.31 N 4.27<br>Found. (%) C 66.20 H 4.18 N 4.10 |
| 87 | 3′-Cl | H | Colorless powder | >300°C | $C_{16}H_{10}ClNO_3$<br>Calcd. (%) C 64.12 H 3.36 N 4.67<br>Found. (%) C 64.06 H 3.26 N 4.66 |
| 88 | 2′,4′-$(Cl)_2$ | Me | Colorless crystals | 222—224°C | $C_{17}H_{11}Cl_2NO_3$<br>Calcd. (%) C 58.64 H 3.18 N 4.02<br>Found. (%) C 58.65 H 3.29 N 3.86 |

| Example No. | Substituent(s) on phenyl | $R^3$ | Appearance | M.p. | Elementary Analysis |
|---|---|---|---|---|---|
| 89 | 2',4'-$(Cl)_2$ | Et | Pale yellow crystals | 175—177°C | $C_{18}H_{13}Cl_2NO_3$<br>Calcd. (%) C 59.68 H 3.62 N 3.88<br>Found. (%) C 59.81 H 3.50 N 3.82 |
| 90 | 2',4'-$(Cl)_2$ | H | Colorless powder | >300°C | $C_{16}H_9Cl_2NO_3$<br>Calcd. (%) C 57.51 H 2.71 N 4.19<br>Found. (%) C 57.32 H 2.90 N 3.94 |
| 91 | 3',4'-$(Cl)_2$ | H | Colorless needles | >300°C | $C_{16}H_9Cl_2NO_3$<br>Calcd. (%) C 57.51 H 2.71 N 4.19<br>Found. (%) C 57.51 H 2.65 N 4.15 |
| 92 | 4'-Me | Et | Pale yellow crystals | 192—193°C | $C_{18}H_{15}NO_3$<br>Calcd. (%) C 73.70 H 5.15 N 4.78<br>Found. (%) C 73.98 H 5.38 N 4.54 |
| 93 | 4'-Me | H | Colorless crystals | >300°C | $C_{17}H_{13}NO_3$<br>Calcd. (%) C 73.11 H 4.69 N 5.02<br>Found. (%) C 73.16 H 4.52 N 4.92 |
| 94 | 2',4'-$(Me)_2$ | Et | Colorless needles | 125—126°C | $C_{20}H_{19}NO_3$<br>Calcd. (%) C 74.74 H 5.96 N 4.36<br>Found. (%) C 74.71 H 5.79 N 4.33 |
| 95 | 2',4'-$(Me)_2$ | H | Colorless powder | 289—291°C | $C_{18}H_{15}NO_3$<br>Calcd. (%) C 73.70 H 5.15 N 4.78<br>Found. (%) C 73.84 H 5.29 N 4.73 |
| 96 | 4'-n-$C_8H_{17}$ | Et | Pale yellow needles | 94—95°C | $C_{26}H_{31}NO_3$<br>Calcd. (%) C 77.00 H 7.71 N 3.71<br>Found. (%) C 77.07 H 7.69 N 3.62 |
| 97 | 4'-n-$C_8H_{17}$ | H | Colorless powder | 202—203°C | $C_{24}H_{27}NO_3$<br>Calcd. (%) C 76.36 H 7.21 N 3.71<br>Found. (%) C 76.57 H 7.21 N 3.82 |
| 98 | 3',4'-$(OMe)_2$ | Et | Colorless needles | 114—116°C | $C_{20}H_{19}NO_5$<br>Calcd. (%) C 67.98 H 5.42 N 3.96<br>Found. (%) C 68.15 H 5.28 N 3.90 |
| 99 | 3',4'-$(OMe)_2$ | H | Colorless needles | 238—240°C | $C_{18}H_{15}NO_5$<br>Calcd. (%) C 66.45 H 4.65 N 4.31<br>Found. (%) C 66.49 H 4.54 N 4.21 |

The compounds as listed hereinabove can, of course, be manufactured by other routes. For example, the compound of Example 83 was prepared as follows.

Thus, 5 grams of beta-(2-chlorophenyl)-beta-(2-ethoxycarbonylimino)-propionic acid p-ethoxyanilide was added to 80 grams of polyphosphoric acid, the mixture was heated to 140 to 150°C for 1 hour, cooled, poured over into ice water, crystals separated out therefrom were collected by filtration, the crystals washed with water, dried and recrystallized from acetone to give 2.3 grams of ethyl 2-(2-chlorophenyl)-4-quinolone-8-carboxylate, pale yellow prisms, melting point 152—153°C.

Example 100

Ethyl 2-(2-chlorophenyl)-4-hydroxyquinoline-8-carboxylate hydrochloride

Ethyl 2-(2-chlorophenyl)-4-quinolone-8-carboxylate (1.98 gram) is dissolved in 50 ml of methanol, stirred at room temperature for 0.5 hour with 10 ml of 40% ethanolic hydrochloric acid, the solvent is evaporated from the solution, and the residue is recrystallized from ethanol to give 1.9 grams of ethyl 2-(2-chlorophenyl)-4-hydroxyquinoline-8-carboxylate hydrochloride, colorless prisms, melting point 196 to 198°C.

Elementary analysis calculated for $C_{18}H_{15}NO_3Cl_2$: C 59.36, H 4.15, N 3.85; Found: C 59.34, H 4.08, H 3.87.

Example 101

Methyl 2-(2-chlorophenyl)-4-quinolone-8-carboxylate

A solution of 10 grams of o-chlorobenzoyl-3-methoxycarbonyl-2-nitroacetophenone in 50 ml of glacial acetic acid was dropped into a solution of 25 grams of stanneous chloride in 50 ml of concentrated hydrochloric acid with stirring. The mixture was then slightly warmed to make it transparent and let stand overnight. Crystals separated out therefrom were collected by filtration, dissolved in warm diluted alkaline solution, and filtered. To the filtrate were added ammonium chloride and ammonia water, the mixture was vigorously stirred, and crystals separated out were collected by filtration. The crystals were washed with water, dried and recrystallized from acetone, to give 1.2 grams of the title product, yellow needles, melting point 166 to 168°C.

Another method is given. Thus, to 8 grams of dimethyl 2-amino-isophthalate was added 9.2 grams of o-chloroacetophenone diethylacetal, the mixture was heated at 140°C for 30 minutes, then at 200°C for additional 20 minutes, and finally heated to reflux for 18 hours. The reaction mixture was cooled, crystals separated out were collected by filtration, and recrystallized from acetone to give 2.8 grams of the title product, melting point 166—168°C.

Still another method is given. Thus, 2.8 grams of o-chlorobenzaldehyde and 50 ml of glacial acetic acid were added to 5.1 grams of 2-amino-3-methoxycarbonyl-omega-(methylsulfinyl)-acetophenone. The mixture was heated at 75 to 80°C for one hour, cooled, poured over into water, crystals separated out therefrom were collected by filtration, and the crystals were washed with water and recrystallized from acetone to give 1 gram of the title product, melting point 166—168°C.

Similarly prepared were the following compounds.

| Example Number | Substituent on Phenyl group | $R^3$ | Appearance | Melting Point |
|---|---|---|---|---|
| 102 | 2'-Cl | Et | Pale Yellow Needles | 164—166°C |
| 103 | 4'-Cl | Et | Colorless Needles | 190—230°C |
| 104 | 2'-Me | Et | Colorless Crystals | 119—123°C |
| 105 | 3',4'-$(Me)_2$ | Et | Pale Yellow Needles | 210—211°C |
| 106 | 4'-iso-Pr | Et | Yellow Needles | 148—149°C |
| 107 | 4'-OMe | Et | Yellow Needles | 192—194°C |
| 108 | 2',4'-$(OMe)_2$ | Et | Pale Yellow Needles | 160—162°C |

Example 109

Beta-Hydroxyethyl 2-(2-chlorophenyl)-4-quinolone-8-carboxylate

2-(2-Chlorophenyl)-4-quinolone-8-carboxylic acid (10 grams) was stirred overnight at 95 to 98°C in a mixture of 10 ml of concentrated sulfuric acid and 100 ml of ethylene glycol. The reaction solution was poured over into water and separated crystals were collected by filtration and then washed with water. After dried, the crystals were recrystallized from acetone to give 8.2 grams of the title compounds, pale yellow prisms, melting point 170 to 171°C.

Example 110

Allyl 2-(2-chlorophenyl)-4-quinolone-8-carboxylate

To 7 grams of potassium salt of 2-(2-chlorophenyl)-4-quinolone-8-carboxylic acid were added 2 ml of allyl bromide and 200 ml of dimethyl formamide, the mixture was stirred at 70 to 80°C for 6 hours, poured into water, the mixture was extracted with chloroform, the solvent was removed from the extract, and the residue was recrystallized from acetone to give 5.6 grams of the title compound, pale yellow needles, melting point 121°C.

Similarly prepared were the following compounds from example numbers 111 to 140.

| Example Number | Substituent on a phenyl group | $R^3$ | Appearance | Melting Point |
|---|---|---|---|---|
| 111 | 2′-Cl | $n-C_3H_7$ | Pale yellow prisms | 138°C |
| 112 | 2′-Cl | $iso-C_4H_9$ | Colorless prisms | 107°C |
| 113 | 2′-Cl | $n-C_6H_{13}$ | Pale yellow columns | 72°C |
| (114 not given) | | | | |
| 115 | 2′-Cl | $(CH_2)_3OH$ | Colorless needles | 169—170°C |
| 116 | 2′-Cl | $(CH_2)_5OH$ | Colorless crystals | 99—105°C |
| 117 | 2′-Cl | $(CH_2)_6OH$ | Colorless crystals | 98—103°C |
| 118 | 2′-Cl | $(CH_2)_2OAc$ | Colorless powder | 122—124°C |
| 119 | 2′-Cl | $(CH_2)_2OPh$ | Pale yellow prisms | 163°C |
| 120 | 2′-Cl | $(CH_2)_2OEt$ | Pale brown crystals | 90—91°C |
| 121 | 2′-Cl | $CH_2CN$ | Yellow needles | 225—227°C |
| 122 | 2′-Cl | $CH_2COCH_3$ | Colorless crystals | 155°C |
| 123 | 2′-Cl | $CH_2COOEt$ | Pale yellow needles | 93.5—94.5°C |
| 124 | 2′-Cl | $(CH_2)_3COOEt$ | Colorless prisms | 102°C |
| 125 | 2′-Cl | $CH_2CH(OH)CH_3$ | Pale yellow crystals | 174—177°C |
| 126 | 2′-Cl | $CH_2CH(OH)CH_2OH$ | Pale brown crystals | 171—173°C |
| 127 | 2′-Cl | $(CH_2)_2O$-iso-Bu | Colorless crystals | 90—93°C |
| 128 | 2′-Cl | $(CH_2)_2O(CH_2)OH$ | Colorless prisms | 133—134°C |
| 129 | 2′-Cl | $(CH_2)_2O(CH_2)_2OEt$ | Colorless crystals | 65—67°C |
| 130 | 2′-Cl | $CH_2COCH_2COOEt$ | Pale brown crystals | 122—123°C |
| 131 | 2′-F | $CH_2CH_2OH$ | Pale yellow crystals | 159—161°C |
| 132 | 3′-Cl | $CH_2CH_2OH$ | Colorless crystals | 186—190°C |
| 133 | 4′-Cl | $CH_2CH_2OH$ | Colorless crystals | 216—218°C |
| 134 | 2′-Me | $CH_2CH_2OH$ | Colorless crystals | 160—163°C |
| 135 | 4′-Me | $CH_2CH_2OH$ | Colorless crystals | 213—216°C |
| 136 | 2′,4′-$Me_2$ | $CH_2CH_2OH$ | Pale yellow plates | 175—176°C |
| 137 | 3′,4′-$Me_2$ | $CH_2CH_2OH$ | Pale yellow needles | 185—187°C |

| Example Number | Substituent on a phenyl group | $R^3$ | Appearance | Melting Point |
|---|---|---|---|---|
| 138 | 4'-OMe | $CH_2CH_2OH$ | Colorless needles | 196—198°C |
| 139 | 2',4'-$(OMe)_2$ | $CH_2CH_2OH$ | Pale yellow needles | 175—177°C |
| 140 | 3',4'-$(OMe)_2$ | $CH_2CH_2OH$ | Pale yellow needles | 212—213°C |

**Claims**

1. 4-quinolone derivatives represented by one of the following formulae:

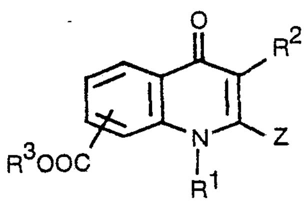

(Ia)

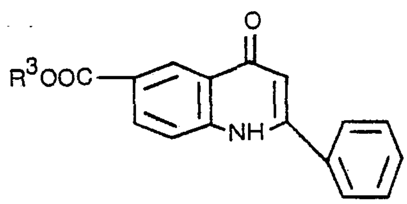

(Ib)

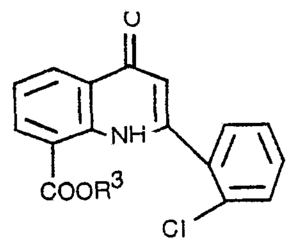

(Ic)

wherein in the formula (Ia),
$R^1$ and $R^2$ are same or different and they are hydrogen, lower alkyl of one to eight carbon chain length or lower alkenyl of up to four carbon chain length; $R^3$ is hydrogen, lower alkyl of 1 to 8 carbon chain length, lower alkyl of 1 to 8 carbon chain length with 1 or 2 hydroxyl group(s), lower alkyl of 3 to 10 carbon chain length with 1 or 2 ether bond(s) or —$(CH_2)_n$A where n is an integer of 1 to 3 and A is acetyl, acetyloxy, cyano or phenoxy; Z is phenyl with or without substituent(s) selected from a group consisting of hydrogen, halogen, lower alkyl of 1 to 8 carbon chain length, lower haloalkyl of 1 to 3 carbon chain length, lower alkoxy or 1 to 4 carbon chain length and $COOR^4$ in which $R^4$ is hydrogen or lower alkyl of 1 to 4 carbon chain length, or Z may be five- or six-membered unsaturated hetero ring radical having nitrogen, oxygen or sulfur as heteroatom;
in the formula (Ib), $R^3$ is phenethyl, and, in the formula (Ic), $R^3$ is allyl, ethoxycarbonylmethyl, ethoxycarbonylpropyl, beta-hydroxyethoxyethyl or ethoxycarbonylacetylmethyl, and salts thereof.

2. Anti-inflammatory agent consisting of one or more of the compounds represented by one of the formulae (Ia), (Ib), (Ic) in claim 1 and salts thereof.

3. Anti-allergic agent consisting of one or more of the compounds represented by one of the formulae (Ia), (Ib), (Ic) in claim 1 and salts thereof.

4. Anti-thrombus agent consisting of one or more of the compounds represented by one of the formulae (Ia), (Ib) and (Ic) in claim 1 and salts thereof.

5. A method of manufacturing a compound represented by one of the formulae (Ia), (Ib) and (Ic) in claim 1, characterized in that a compound of the formula (II) or tautomer thereof

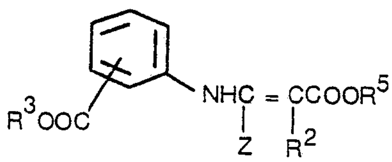

(II)

in which $R^2$, $R^3$ and Z have the same meanings as already defined; $R^5$ is lower alkyl is subjected to a ring closure reaction.

6. A method of manufacturing a compound represented by one of the formulae (Ia), (Ib) and (Ic) in claim 1, characterized in that a compound of the following formula

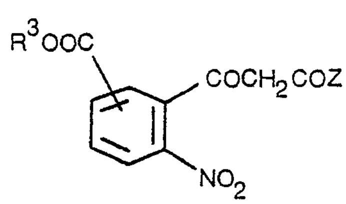

in which $R^3$ and Z have the same meanings as already defined is subjected to a reduction followed by a ring closure reaction.

7. A method of manufacturing a compound represented by one of the formulae (Ia), (Ib) and (Ic) in claim 1, characterized in that a compound of the following formula

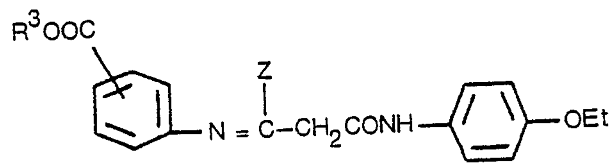

or tautomer thereof is subjected to a ring closure reaction.

8. A method of manufacturing a compound represented by one of the formulae (Ia), (Ib) and (Ic) in claim 1, characterized in that a compound of the following formula

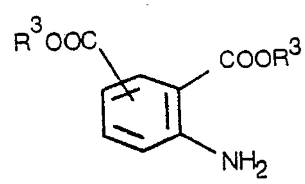

in which $R^3$ has the same meaning as already defined, is reacted with a compound of the following formula

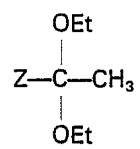

(in which Z has the same meaning as already defined).

9. A method of manufacturing a compound represented by one of the formulae (Ia), (Ib) and (Ic) in claim 1, characterized in that a compound of the following formula

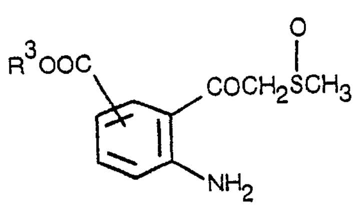

in which $R^3$ has the same meaning as already defined is reacted with a compound of a formula ZCHO in which Z has the same meaning as already defined.

**Patentansprüche**

1. 4-Chinolon-Derivate, die durch eine der folgenden Formeln wiedergegeben werden:

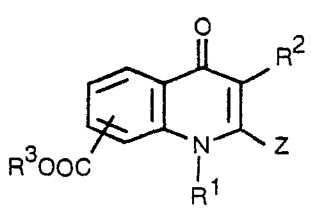

(Ia)

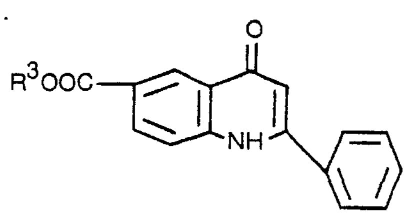

(Ib)

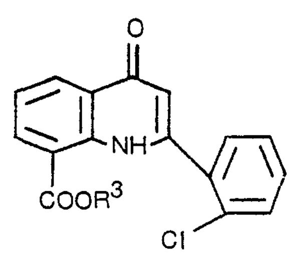

(Ic)

worin in der Formel (Ia) $R^1$ und $R^2$ gleich oder verschieden sind und Wasserstoff, ein niedriges Alkyl mit 1 bis 8 Kohlenstoffatomen in der Kette oder ein niedriges Alkenyl mit bis zu 4 Kohlenstoffatomen in der Kette sind; $R^3$ Wasserstoff, ein niedriges Alkyl mit 1 bis 8 Kohlenstoffatomen in der Kette, ein niedriges Alkyl mit 1 bis 8 Kohlenstoffatomen in der Kette und mit 1 oder 2 Hydroxylgruppen, ein niedriges Alkyl mit 3 bis 10 Kohlenstoffatomen in der Kette mit 1 oder 2 Etherbindungen oder $-(CH_2)_n$ A ist, worin n eine ganze Zahl zwischen 1 und 3 und A Acetyl, Acetyloxy, Cyan oder Phenoxy ist; Z Phenyl mit oder ohne Substituenten,

ausgewählt aus einer Gruppe, die aus Wasserstoff, Halogen, einem niedrigen Alkyl mit 1 bis 8 Kohlenstoffatomen in der Kette, einem niedrigen halogen-substituierten Alkyl mit 1 bis 3 Kohlenstoffatomen in der Kette, einem niedrigen Alkoxy mit 1 bis 4 Kohlenstoffatomen in der Kette und $COOR^4$ besteht, ist, wobei $R^4$ Wasserstoff oder ein niedriges Alkyl mit 1 bis 4 Kohlenstoffatomen in der Kette ist oder Z ein fünf- oder sechsgliedriges ungesättigtes Heteroringradikal mit Stickstoff, Sauerstoff oder Schwefel als Heteroatom ist; in der Formel (Ib) $R^3$ Phenethyl ist und in der Formel (Ic) $R^3$ Allyl, Ethoxycarbonylmethyl, Ethoxycarbonylpropyl, beta-Hydroxyethoxyethyl oder Ethoxycarbonylacetylmethyl ist, sowie Salze davon.

2. Entzündungshemmendes Mittel, bestehend aus einer oder mehreren Verbindungen, welche durch eine der Formeln (Ia), (Ib), (Ic) gemäß Anspruch 1 und deren Salze wiedergegeben werden.

3. Antiallergisches Mittel, bestehend aus einer oder mehreren Verbindungen, welche durch eine der Formeln (Ia), (Ib), (Ic) gemäß Anspruch 1 und deren Salze wiedergegeben werden.

4. Antithrombusmittel, bestehend aus einer oder mehreren Verbindungen, welche durch eine der Formeln (Ia), (Ib), (Ic) gemäß Anspruch 1 und deren Salze wiedergegeben werden.

5. Verfahren zur Herstellung einer Verbindung, welche durch eine der Formeln (Ia), (Ib) und (Ic) gemäß Anspruch 1 wiedergegeben wird, dadurch gekennzeichnet, daß eine Verbindung der Formel (II) oder ein Tautomeres davon,

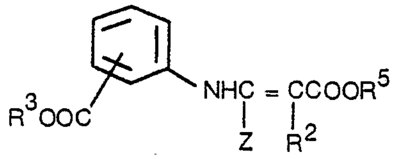

(II)

worin $R^2$, $R^3$ und Z die gleiche Bedeutung haben, wie sie bereits definiert worden ist und $R^5$ ein niedriges Alkyl ist, einer Ringschlußreaktion unterworfen wird.

6. Verfahren zur Herstellung einer Verbindung, welche durch eine der Formeln (Ia), (Ib) und (Ic) gemäß Anspruch 1 wiedergegeben wird, dadurch gekennzeichnet, daß eine Verbindung der folgenden Formel

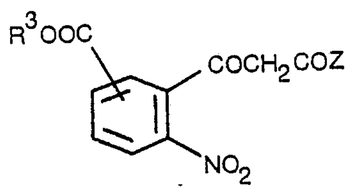

in welcher $R^3$ und Z die gleiche Bedeutung haben, wie sie bereits definiert worden ist, einer Reduktion unterworfen werden, gefolgt von einer Ringschlußreaktion.

7. Verfahren zur Herstellung einer Verbindung, welche durch eine der Formeln (Ia), (Ib) und (Ic) gemäß Anspruch 1 wiedergegeben wird, dadurch gekennzeichnet, daß eine Verbindung der folgenden Formel

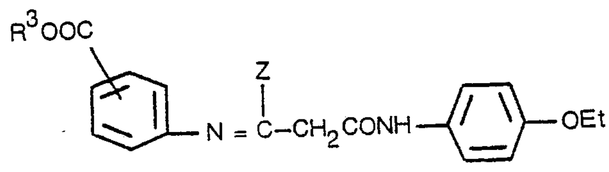

oder ein Tautomeres davon einer Ringschlußreaktion unterworfen wird.

8. Verfahren zur Herstellung einer Verbindung, welche durch eine der Formeln (Ia), (Ib) und (Ic) gemäß Anspruch 1 wiedergegeben wird, dadurch gekennzeichnet, daß eine Verbindung der folgenden Formel

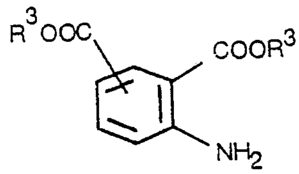

in der $R^3$ die gleiche Bedeutung hat, wie sie bereits definiert worden ist, mit einer Verbindung der folgenden Formel umgesetzt wird.

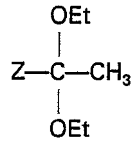

(in dem Z die gleiche Bedeutung hat, wie sie vorstehend definiert ist).

9. Verfahren zur Herstellung einer Verbindung, welche durch eine der Formeln (Ia), (Ib) und (Ic) gemäß Anspruch 1 wiedergegeben wird, dadurch gekennzeichnet, daß eine Verbindung der folgenden Formel

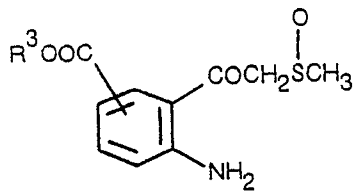

in der $R^3$ die gleiche Bedeutung hat, wie sie bereits definiert worden ist, mit einer Verbindung der Formel ZCHO umgesetzt wird, in der Z die gleiche Bedeutung hat, wie sie bereits definiert worden ist.

**Revendications**

1. Dérivés de la 4-quinolone répondant à l'une des formules suivantes:

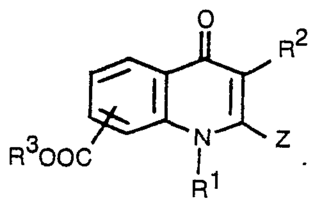

(Ia)

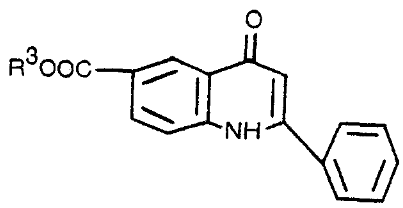

(Ib)

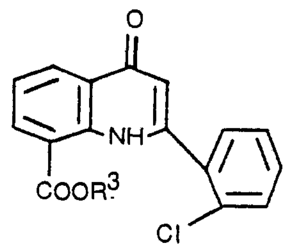

(Ic)

dans lesquelles, dans la formule Ia
$R^1$ et $R^2$ sont identiques ou différents et sont l'hydrogène, un groupe alkyle inférieur en C1 à C8 ou alcényle inférieur ayant jusqu'à quatre atomes de carbone; $R^3$ est l'hydrogène, un groupe alkyle inférieur en C1 à C8, un groupe alkyle inférieur en C1 à C8 avec un ou deux groupes hydroxyle, un groupe alkyle inférieur en C3 à C10 avec une ou deux liaisons éther ou $—(CH_2)_nA$, où n est un entier de 1 à 3 et A est un groupe acétyle, acétyloxy, cyano ou phénoxy; Z est un groupe phényle avec ou sans substituant, choisi parmi l'hydrogène, les halogènes, un groupe alkyle inférieur en C1 à C8, un groupe haloalkyle inférieur en C1 à C3, un groupe alcoxy inférieur en C1 à C4 et $COOR^4$, où $R^4$ est l'hydrogène ou un groupe alkyle inférieur en C1 à C4, ou bien Z peut être un radical hétérocyclique insaturé à cinq ou six maillons ayant l'azote, l'oxygène ou le soufre comme hétéroatome; dans la formule Ib, $R^3$ est un groupe phénéthyle, et dans la formule Ic $R^3$ est un groupe allyle, éthoxycarbonylméthyle, éthoxycarbonylpropyle, bétahydroxyéthoxyéthyle ou éthoxycarbonylacétylméthyle, et leurs sels.

2. Agent anti-inflammatoire constitué d'un ou plusieurs des composés répondant à l'une des formules Ia, Ib, Ic de la revendication 1, et de leurs sels.

3. Agent anti-allergique constitué d'un ou plusieurs des composés répondant à l'une des formules Ia, Ib, Ic de la revendication 1 et de leurs sels.

4. Agent anti-thrombus constitué d'un ou plusieurs des composés répondant aux formules Ia, Ib et Ic de la revendication 1 et de leurs sels.

5. Procédé de préparation d'un composé répondant à l'une des formules Ia, Ib, Ic de la revendication 1, caractérisé en ce que l'on soumet à une réaction de fermeture de cycle un composé répondant à la formule II ou un tautomère de celui-ci

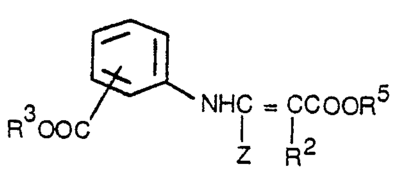

(II)

formule dans laquelle $R^2$, $R^3$ et Z ont les mêmes significations que précédemment; $R^5$ est un groupe alkyle inférieur.

6. Procédé de préparation d'un composé répondant à une des formules Ia, Ib et Ic de la revendication 1, caractérisé en ce que l'on soumet à une réduction suivie d'une réaction de fermeture de cycle un composé répondant à la formule suivante

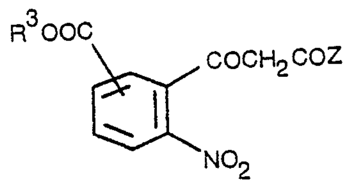

dans laquelle $R^3$ et Z ont les mêmes significations que précédemment.

7. Procédé de préparation d'un composé répondant aux formules Ia, Ib et Ic de la revendication 1, caractérisé en ce que l'on soumet à une réaction de fermeture de cycle un composé répondant à la formule suivante

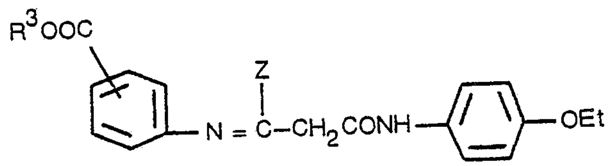

ou un tautomère de celui-ci.

8. Procédé de préparation d'un composé répondant à l'une des formules Ia, Ib et Ic de la revendication 1, caractérisé en ce que l'on fait réagir un composé répondant à la formule suivante

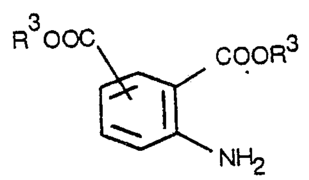

dans laquelle $R^3$ a la même signification que précédemment, avec un composé répondant à la formule suivante

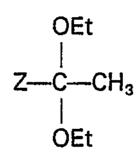

(dans laquelle Z a la même signification que précédemment).

9. Procédé de préparation d'un composé répondant à l'une des formules Ia, Ib et Ic de la revendication 1, caractérisé en ce que l'on fait réagir un composé répondant à la formule suivante

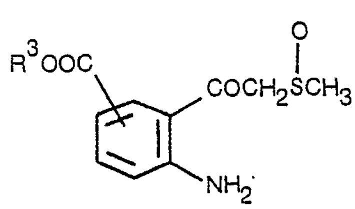

dans laquelle $R^3$ a la même signification que précédemment, avec un composé répondant à la formule ZCHO, dans laquelle Z a la même signification que précédemment.